# EUROPEAN PATENT APPLICATION

(11) **EP 1 118 333 A1**
(43) Date of publication of application: **25.07.2001**
(21) Application number: 00100886.1
(22) Date of filing: 18.01.2000
(51) Int. Cl.: A61K 47/14, A61K 47/18, A61K 47/36

(54) **Compositions with enhanced oral bioavailability**

(71) Applicant: Eurand International S.p.A., 20121 Milano (IT)
(72) Inventor: Carreno-Gomez, Begona, London WC1N 2DL (GB); Duncan, Ruth, London NW1 5TF (GB)
(74) Representative: Gervasi, Gemma, Dr.

(57) **Abstract**

The present invention describes the use of polysaccharides, surfactants, and dendrimers as bioabvailability enhancers for oral pharmaceutical compositions. These substances exhert an inhibitory action of the gastrointestinal pump efflux proteins, such as the P-glycoprotein and the MDR protein, responible for poor drug bioavailability and multidrug resistance. The formulations herein described comprise a medicinally active substance in association with said polysaccharides, surfactants, and dendrimers. They are well tolerated, are not absorbed by the gastrointestinal tract, and increase the bioavailability and the activity of orally administered medicaments, like e.g. antineoplastic, antiviral, antibiotic, or antidepressant medicaments.

## Description

### Field of the invention

The present invention relates to the use of certain compounds to improve bioavailability, especially oral bioavailability, of medicinally active substances and/or to inhibit the effect of so-called "efflux pump" systems such as p-glycoprotein and multidrug resistance associated proteins (MRP). Methods and pharmaceutical compositions suitable for putting the invention into effect are also described.

### Prior art

The oral route is often the most convenient for drug administration, but unfortunately many drugs are not orally active due to their poor bioavailability. Many drugs also show highly variable oral bioavailability.

The bioavailability of many medicinally active substances is reduced by the action of so-called "efflux pump" proteins which actively eject such substances from the cell to give rise, for example, to the so-called "multi-drug resistance" (MDR) effect. One of the best known such "efflux pump" enzymes is P-glycoprotein (P-gp).

P-glycoprotein is a 170-180 KDa transmembrane protein member of the ABC family and is responsible for poor oral bioavailability of many pharmaceuticals such as antibiotics, anticancer agents, antidepressants etc and for multidrug resistance (MDR). Although P-glycoprotein is over-expressed in cancer cells, it has also been found in healthy tissues including the blood brain barrier, pancreas, liver and the intestinal tract. P-glycoprotein acts as an "efflux pump" which binds to drugs, especially lipohilic drugs, within the outer leaflet of the membrane and flips back into the lumen (Hunter, J., and Hirst, B.H. (1997). Adv Drug. Deliv. Rev. 25, 129-157.). Its normal role has been considered to be a detoxifying system in epithelial cells by stopping toxins or xenobiotics from entering into the cell. Its expression varies among different individuals which in turn is responsible for patient variability.

Although membrane located efflux proteins are well known as a factors contributing to the acquired multidrug resistance syndrome arising in many cancer patients after repeated chemotherapy, it has only recently been reaslised that p-gp is also found in the normal tissue such as small intestine, colon, liver and endothelial cells in the blood brain barrier. The presence of such efflux proteins e.g. p-glycoprotein (p-gp), in the GI tract, especially in the small intestine and colon, contributes to the poor bioavailability of many natural product drugs (including the anticancer agents vinblastine and doxorubicin). Many chemotherapeutic agents given orally can not show antitumour activity due to poor bioavailability and their inability to enter GI tissue also leads.
Although many systems have been described to ihnibit p-gp in mdr tumours, little has been done to target efflux proteins in the GI tract to an inherent resistance to GI cancers to treatment by chemotherapy.
A number of workers have investigated possible low-molecular weight inhibitors of p-glycoprotein.

Dale *et al* in British Journal of Cancer (1996) describe the use of the methylbenzamide compound XR9051 to reduce P-glycoprotein-mediated multidrug resistance.

Molnar *et al* in Anticancer Research 17: 481-486 (1997) describe the effects of certain substituted chlorpromazines on multidrug resistant tumor cells.

Low molecular weight inhibitors of efflux pump proteins effectively block p-gp-mediated drug transport (including promotion of GI transport in vitro model systems).
Such low molecular weight compounds have a major disadvantages for commercial development. They are inherently pharmacologically active and as they readily distribute throughout the body their co-administration (with anticancer or other agents) leads to considerable toxicological problems. This has already been demonstrated clinically.

It has now surpisingly been found that certain compounds can act as oral bioavailability enhancers and can be used to improve the bioavailability of medicinally active substances and/or reduce or alleviate the multidrug resistance phenomenon in animals, including mammals such as humans. The compounds which have been found, according to the present invention, to have this surprising utility are selected from polysaccharides, surfactants and dendrimers.

The macromolecular inhibitors of efflux proteins described here have the advantage that they largely remain within the GI tract following oral administration and thus can promote oral bioavailability without increasing toxicity.

Without wishing to be bound to any particular theory of mode of action, it is believed that the present bioavailability enhancers may exert their effect by inhibiting "efflux pump" active transport enzymes and, in particular, the P-glycoprotein or MRP mediated efflux of medicinally active substances from the cell. Alternatively or in addition the bioavailability enhancers may act by "shielding" medicinally active substances from metabolism linked to CYP3A (an enyme which constitutes 70% of total P450 activity in the human intestine and is known to be responsible for breakdown of a large number of medicinally active substances in the gut).

Accordingly, in a first aspect the present invention provides a bioavailability enhancer in the form of a polysaccharide, surfactant or dendrimer for use to improve the oral bioavailability of a medicinally active substance.

In a further aspect the invention provides such a bioavailability enhancer for use to improve the oral bioavailability of a medicinally active substance.

In a further aspect the invention provides a bioavailability enhancer in the form of a polysaccharide, surfactant or dendrimer for use to inhibit an efflux pump system of the type causing ejection of medicinally active substances from the cell. Suitably the efflux pump system is P-glycoprotein (P-gp) or MRP.
In a further aspect the invention provides a bioavailability enhancer in the form of a polysaccharide, surfactant or dendrimer for use to prevent or alleviate the multi-drug resistance (MDR) effect.

In a further aspect the invention provides the use of a bioavailability enhancer in the form of a polysaccharide, surfactant or dendrimer in the manufacture of a medicament to improve the oral bioavailability of a medicinally active substance.

In a further aspect the invention provides the use of a bioavailability enhancer in the form of a polysaccharide, surfactant or dendrimer in the manufacture of a medicament to improve the oral bioavailability of a medicinally active substance.

In a further aspect the invention provides the use of a bioavailability enhancer in the form of a polysaccharide, surfactant or dendrimer in the manufacture of a medicament to inhibit an efflux pump system of the type causing ejection of medicinally active substances from the cell. Suitably the efflux pump system is P-glycoprotein (P-gp), MRP, or other efflux pump system such as the fluochrome efflux system or the methotrexate efflux system.

In a further aspect the invention provides the use of a bioavailability enhancer in the form of a polysaccharide, surfactant or dendrimer in the manufacture of an oral medicament to alleviate multi-drug resistance (MDR).

Suitably the medicinally active substance is a compound which is a substrate for an efflux pump system of the type causing ejection of medicinally active substances from the cell. Suitably the medicinally active substance is a substrate for P-glycoprotein (P-gp), MRP, or other efflux systems as mentioned above.

The present invention also provides a method for improving the oral bioavailability of a medicinally active substance comprising administering the medicinally active substance to an animal in need thereof and simultaneously, sequentially or separately administering an effective amount of a bioavailability enhancer in the form of a polysaccharide, surfactant or dendrimer (or a combination thereof).

The present invention also provides a method for inhibiting an efflux pump system of the type causing ejection of medicinally active substances from a cell comprising administering an effective amount of a bioavailability enhancer in the form of a polysaccharide, surfactant or dendrimer to the cell. Suitably the cell is a gut cell.

The present invention also provides a method of preventing, reducing or overcoming the multi-drug resistance (MDR) effect in an animal comprising administering a medicinally active substance to an animal in need thereof and simultaneously, sequentially or separately administering an effective amount of a bioavailability enhancer in the form of a polysaccharide, surfactant or dendrimer.

The present invention also provides a method of treating cancer comprising administering an anticancer agent to an animal in need thereof and simultaneously, sequentially or separately administering a bioavailability enhancer in the form of a polysaccharide, surfactant or dendrimer in an amount effective to inhibit P-glycoprotein (P-gp), MRP, the flurochrome efflux system or the methotrexate efflux system. The administration is oral.

The present invention also provides a pharmaceutical composition for oral administration, comprising a bioavailability enhancer in the form of a polysaccharide, surfactant or dendrimer in an amount effective to inhibit P-gp and a medicinally active substance. In one embodiment the composition is enteric coated to prevent or reduce degradation of the polysaccharide material before it reaches the gut in use.

Suitably the bioavailability enhancer is used therapeutically in an amount of from 5 mg dose/kg body weight/day to about 5 g dose/kg body weight/day. Suitably the enhancer is used at or below its WHO LD₅₀ value. However, having regard to patient variability individual doses must be within the discretion of the physician.

Preferably the medicinally active substance with which the bioavailability enhancer is used is a compound which is a substrate for an efflux pump system of the type causing ejection of medicinally active substances from the cell, and preferably the medicinally active substance is a substrate for P-glycoprotein (P-gp), or MRP.

In one embodiment the medicinally active substance may be an anti-tumour/anti-neoplastic agent such as vincristine, doxorubicin, vinblastine, methotrexate, cisplatin, daunorubicin, adriamycin or mitomycin C.

Alternatively, the medicinally active substance may be, for example, an antibiotic/antibacterial agent such as amikacin, tetracycline, ampicillin, cefotaxime or piperacillin; an antiviral agent such as acyclovir, rifampicin; an antifungal agent such as miconazole, ketoconazole, clotrimazole, nystatin or butaconazole; an antidepressant agent such as imipramine and clomipramine or a hormone or hormone derivative such as cortisol, prednisone, progesterone or clomiphene.

The invention further provides a pharmaceutical composition for oral administration as defined above which comprises a polysaccharide in the form of a xanthan gum, gellan gum or an alginate salt (preferably an alkali metal alginate, such as sodium alginate) and a medicinally active substance in the form of vincristine or doxorubicin.

Suitably the pharmaceutical composition may be enteric coated to prevent or reduce degradation of the bioavailability enhancer before it reaches the gut. This ensures that the bioavailability enhancer has the maximum effect at its desired point of action in the gut.

Suitably, the medicinally active substance and the bioavailability enhancer are present together in the composition. For example, the composition may take the form of capsules containing a homogeneous solution or suspension of the medicinally active substance and the bioavailability enhancer. Presence of the bioavailability enhancer in solution or suspension allows rapid and efficient dispersal of the bioavailability enhancer in the gut, thus ensuring the maximum bioavailability-enhancing effect on the cells lining the gut wall.

Where a polysaccharide is used as an oral bioavailability enhancer this may, for example, be an anionic polysaccharide. Preferably the polysaccharide used in the invention has carboxylic acid functional groups or salts thereof in at least some of its monomer residues. Suitably the polysaccharide comprises D-mannosyluronic acid, L-gulosyluronic, D-glucose and/or D-glucuronic acid monomers and optionally also D-mamose, D-mannuronic acid and/or D-mannose monomers.

Suitably where a polysaccharide is used as an oral bioavailability enhancer this may be a hydrophilic colloidal polysaccharide, suitably a gum such as xanthan gum or gellan gum. Alternatively it may be a polysaccharide gel such as an alkali metal alginate, for example sodium alginate.

Preferred gums are xanthan gum and gellan gum. Xanthan gum is an anionic polysaccharide extracted from the *xanthonomas compestris* bacteria and consists of a cellulose backbone (D-glucose in β(1-4) linkage) attached to side groups of D-mamose, D-glucuronic acid and D-mannose, every other β-D-glucose residue. It has a molecular weight (MWt) of ~2x10⁶ Da. It is used as a stabilising agent, suspending agent, and viscosity-increasing agent. Its toxicity profile has been set by the WHO at up to 10 mg/kg body weight (Godet, 1973). In rats (orally) the LD₅₀ is > 45g/kg.
Gellan gum is a polysaccharide obtained from the *pseudonomas elodea* bacteria. Its structure consists of a β(1-4) linkage of D-glucose, D-glucuronic acid and rhamnose and **it has a** molecular weight of ~0.5x10⁶ Da. It is known for use as a stabiliser and texturiser. LD₅₀ orally in rats is >5 g/kg.

Preferred sodium alginates are Ascophyllum and Flavicam. Ascophyllum is extracted from *Ascophyllum nodosum* and is high in mannuronic acid. Flavicam comes from *Lessonia flavicams* and has a low mannuronic acid:guluronic acid ratio (information provided by the supplier). Molecular weight (MWt) is 690 and 797 KDa for ascophyllum and flavicam respectively, as determined by size exclusion-HPLC (Al- Shamkhani, 1993). Both alginates are used for gel forming and cell entrapment. The WHO recommends 25mg/kg/day (LD₅₀ i.p. in rats 1.6 g/kg)

Preferred polysaccharides include dextran and fucoidan. Dextran is a neutral polysaccharide extracted from the *leuconstoc sp*. Bacteria. It is composed of D-glucose in α(1-6) linkage, branch linkages are α(1-4), α(1-2) and α(1-3). It has been used as a therapeutic agent in restoring blood volume in mass casualties (Kost and Goldbart, 1993. Kaplan and Park (1995) described crosslinked dextran hydrogel to control release of active proteins.

Fucoidan is a naturally occurring polysaccharide, obtained from brown seaweed, composed of α (1-2)-linked units of 4-sulpharyl-L-fucose with some branching or a second sulphate at the C-3 position. The polymer used in this study is of *Mw=* >853,000 determined by size exclusion-HPLC (Al-Shamkani, 1993). They have been shown to possess anticoagulant properties and also to mediate eosinophil recruitment *in vivo* (Teixeira and Hellewell, 1997).

Suitably where a polysaccharide is used this has an average molecular weight of at least 1 x 10⁴ Da, preferably at least 1 x 10⁵ Da, for example from approximately 0.1 x 10⁶ Da to 1 x 10⁷ Da, for example 0.5 x 10⁶ Da to 2 x 10⁶ Da. The polysaccharides may be used alone or in combinations.

Suitably if a surfactant is used it is a polyoxyethylene alkyl ether of the formula I:

Alk―(OCH₂CH₂)ₙ-OH (I)

wherein n is an integer from 5 to 16 and Alk is a C₄₋₁₈ alkyl group.

Preferably the surfactant is a compound wherein n is 9 and Alk is a lauryl (C₁₂ alkyl) group (also known as polidocanol or laureth 9).

Polidocanol is polyoxyethylene-9-lauryl ether. Polyoxyethylene alkyl ethers consist of a series of polyoxythelene glycol ethers of n-alcohols (lauryl in this case). They are non-ionic surfactants produced by the polyethoxylation of linear fatty alcohols. Products tend to be mixtures of polymers of slightly varying molecular weights and the numbers used to describe polymer lengths are average values. Although used at high concentrations (< 20%) they have been shown to cause irritation, the oral LD₅₀ in rats ranges between 2-4 g/kg body-weight. Polidocanol is known for use as an emusifying, solubilising and wetting agent.

Where a dendrimer is used as a bioavailability enhancer this is preferably a compound of the fomula II or a pharmaceutically acceptable salt thereof: 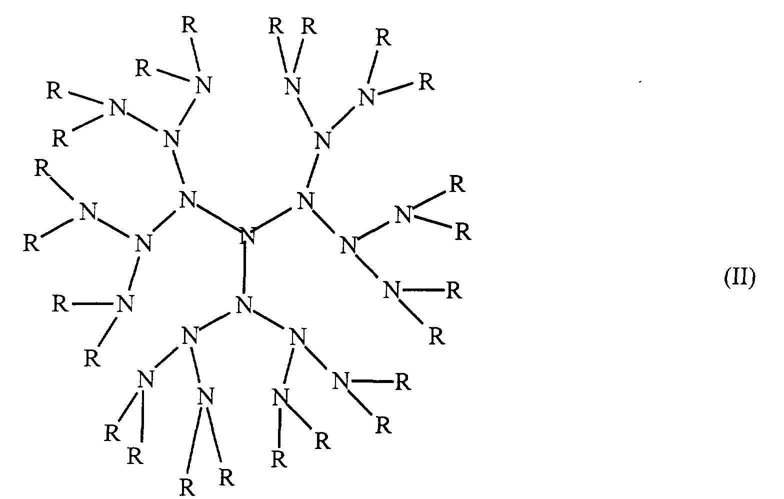 wherein each R moiety is independently hydrogen, an -NH₂ group or a -COOH group; or a compound of the fomula III or a pharmaceutically acceptable salt thereof: 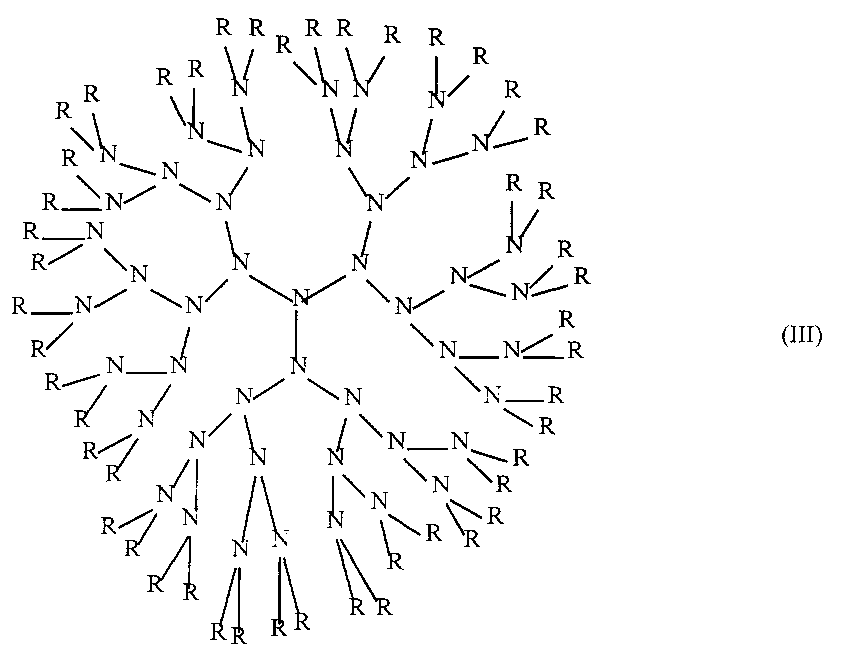 wherein each R moiety is independently hydrogen, an -NH₂ group or a -COOH group. Dendrimers of formula II or III wherein all the R moieties are either hydrogen or -NH₂ groups are known as cationic dendrimers and dendrimers of formula II or III wherein all the R moieties are either hydrogen or -COOH groups are known as anionic dendrimers.

Suitably the dendrimer may be a generation 3 cationic dendrimer of the formula IV: 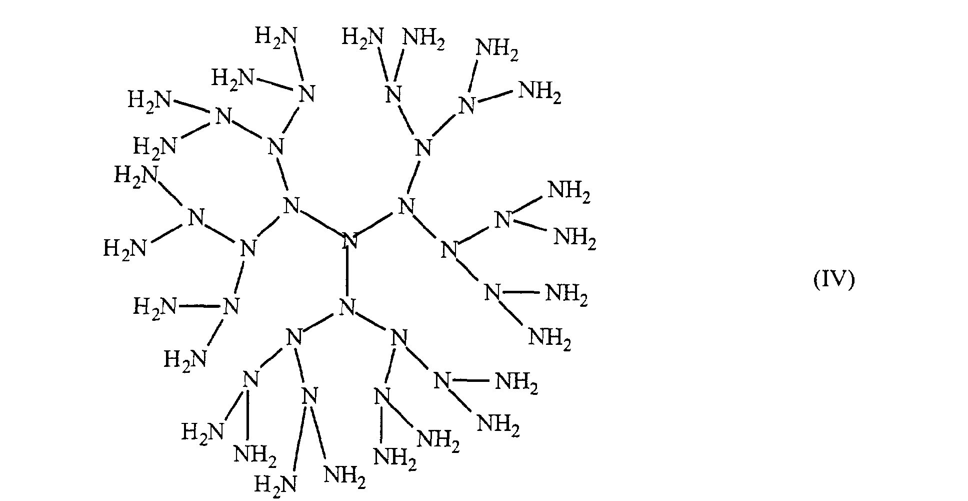 or a pharmaceutically acceptable salt thereof.

Alternatively the dendrimer may be a generation 3.X dendrimer based on Formula II above but with 0.X of the hydrogen atoms replaced by further -NH₂ or -COOH groups; for example a generation 3.5 anionic dendrimer with half of the hydrogen atoms of Formula II replaced by -COOH groups.

Preferred dendrimers are Polyamidoamine Starburst® Dendrimers such as a cationic dendrimer of Generation 3 (G3; 6,909 Da) and/or an anionic dendrimer of Generation 3.5 (G3.5; 12,419 Da). These macromolecules have been reported as potential drug carriers (Malik et al., 1997. Proceed Int. Symp.Control. Rel. Bioac. Mater, 24, 107). The cationic dendrimer G3 LD₅₀ has been reported to > 95 mg/kg i.p. x 3 days (Malik et al., J. Control. Rel., in press), and the anionic dendrimer G3.5 LD₅₀ in B16 F10 cell line has been reported to be > 0.1 mg/ml (Malik et al., J. Control. Rel., in press).

Solid form compositions suitable for use in the invention may include powders, granules, tablets, capsules (e.g. hard and soft gelatine capsules), suppositories and pessaries. A solid carrier may be included such as, for example, one or more substances which may also act as flavouring agents, lubricants, solubilisers, suspending agents, fillers, glidants, compression aids, binders or tablet-disintegrating agents; an encapsulating material may also be used. In powders the carrier may be a finely divided solid which is in admixture with the finely divided active ingredient. In tablets the active ingredient may be mixed with a carrier having the necessary compression properties in suitable proportions and may be compacted in the shape and size desired. The powders and tablets preferably contain up to 99%, e.g. from 0.03 to 99%, preferably 1 to 80% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

The term "composition" includes the formulation of an active ingredient with encapsulating material as carrier to give a capsule in which the active ingredient and polysaccharide (with or without other carriers) is surrounded by the carrier, which is thus in association with it. Similarly cachets are included.

Liquid form compositions include, for example, solutions, suspensions, emulsions, syrups, elixirs and pressurised compositions. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilisers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above, e.g. cellulose derivatives, preferably sodium carboxymethyl cellulose solution), alcohols, (e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers may be used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged composition, for example packeted powders, vials, ampoules, prefilled syringes or sachets containing liquid. The unit dosage form can be, for example, a capsule or tablet itself, or it can be the appropriate number of any such compositions in package form. The quantity of the active ingredient in unit dose of composition may be varied or adjusted from 0.5 mg or less to 750 mg or more, according to the particular need and the activity of the active ingredient.

The present invention will now be further described with reference to the following nonlimiting examples.

### Example 1 (Comparative)

### Example 1: Preparation of everted rat intestinal sacs, establishment of sac viability and use to monitor the transfer of [³H]Vinblastine and [¹⁴C]Doxorubicin in the presence and absence of verapamil

Adult male Wistar rats (200-300 g; 11-12 weeks old) were starved overnight, sacrificed by cervical dislocation and the small intestine removed and washed through three times with saline (0.9% NaCl solution) at room temperature. The intestine was immediately placed in 37°C, oxygenated (O₂/CO₂, 95%:5%) medium 199. The intestine was everted on a glass rod (2.5 mm in diameter) and one end clamped before filling the with fresh, oxygenated medium using a 50 ml plastic syringe. The intestine was then sealed with a second clamp. Small sacs (2.5-3 cm in length) were then tied using silk braided sutures. Each sac was placed in a 50 ml Erlenmeyer flask containing 9.0 ml of oxygenated media at 37°C.

To study the tissue uptake and serosal transfer of [³H]vinblastine or [¹⁴C]doxorubicin in presence or absence of verapamil (0-40 µg/ml) these substrates were added to the flask in 1 ml of culture medium to give a final concentration of 1ng/ml. Other concentrations of anticancer agents were also studied, (0-1 ng/ml)[³H]vinblastine and (0-300 ng/ml) [¹⁴C] doxorubicin.
The flasks were stopped using gas-tight silicone bungs. Sacs were incubated at 37°C in a Grant Instruments (Cambridge, U.K., 5540-5) shaking water bath (70 beats/min). At the desired time points, sacs were removed, washed three times in saline and blotted dry. Sacs were then weighed and the serosal contents (1 ml) drained into small tubes. Sacs were re-weighed after draining to calculate accurately the volume inside each sac. The sacs were then digested individually in 5 ml of 5 M NaOH at 37°C overnight, and then samples of the tissue digest (0.8 ml) were neutralised with 5 M HCl (0.2 ml). The protein content of the digest was determined by the Lowry method as modified by Peterson (Peterson GL (1983) Determination of Total Protein. Methods in Enzymology. 91: 95-119).

To estimate radioactivity, scintillation fluid (4 ml) was added to samples of the incubation media (2 x 1 ml), the serosal fluid (1 ml) and tissue digest (1 ml), was added and samples were counted using a Beckham scintillation counter. The drug present in tissue was expressed as ng /mg of total sac protein and the serosal transport as ng transferred /mg of total sac protein.

In order to verify the integrity of the sacs, glucose was measured both in the incubation medium and in the sac contents using a modification of the method by Dahlqvist (Dahlqvist A (1968) Assay of intestinal disaccharidases. Anal. Biochem. 22: 99-107). Briefly, 20 µl of sample were incubated for 45 minutes with 1 ml of glucose oxidase reagent (0.2% Triton-X100 (w/v in ethanol)), 10µg/ml O-dianisidine-HCI, 1 µg/ml peroxidase, 200 µg/ml glucose oxidase in 0.5 M Tris/HCl, pH 7.2). The reaction was stopped by the addition of 2 ml of 5 M HCl, and the absorbance measured at 525 nm.

**Results:-** Results are shown in Tables 1 to 10 below. With the exception of [³H]vinblastine concentrations higher than 1 ng/ml or verapamil concentrations of 40 µg/ml when combined with 1ng/ml [³H]vinblastine, glucose was actively transported which indicated good tissue viability.
The serosal transfer of [³H]vinblastine increased linearly with time throughout, whereas the transfer of [¹⁴C]doxorubicin was only linear for 90 min, after which time levels plateaued. Addition of verapamil (40 ng/ml) significantly (p<0.05, unpaired student T-test) increased the tissue uptake and serosal transfer of both compounds (Tables 1 and 2). The enhancement of [³H]vinblastine uptake was found to be 13 fold after 1 hour in the presence of 40 ng/ml verapamil (Tables 3 and 5); whereas the same inhibitor concentration increased [¹⁴C]doxorubicin uptake and accumulation into the tissue by only 2 fold (Tables 4 and 6). When tissue levels of uptake are carefully examined, it can be seen that [¹⁴C]doxorubicin was also more readily metabolised in the enterocytes than [³H]vinblastine. verapamil had a significant effect on uptake of both drugs.

**Table 1.**

| **[**^{**3**}**H]Vinblastine (60 min) uptake in rat everted gut sacs (n=6 ± SEM)** | | |
|---|---|---|
| **Concn (µg/ml)** | **Serosal transfer (ng/mg of protein)** | **Tissue uptake (ng/mg of protein)** |
| 0 | 0.000 ± 0.000 | 0.000 ± 0.000 |
| 0.1 | 0.000 ± 0.000 | 0.009 ± 0.000 |
| 0.3 | 0.001 ± 0.000 | 0.011 ± 0.001 |
| 0.7 | 0.002 ± 0.001 | 0.017 ± 0.001 |
| 1.0 | 0.003 ± 0.001 | 0.029 ± 0.007 |

**Table 2.**

| **[**^{**14**}**C]Doxorubicin (60 min) uptake in rat everted gut sacs (n=6 ± SEM)** | | |
|---|---|---|
| **Concn (ng/ml)** | **Serosal transfer (ng/mg of protein)** | **Tissue uptake (ng/mg of protein)** |
| 0 | 0.001 ± 0.008 | 0.003 ± 0.003 |
| 1 | 0.0017 ± 0.002 | 0.007 ± 0.005 |
| 3 | 0.029 ± 0.005 | 0.082 ± 0.015 |
| 10 | 0.193 ± 0.028 | 0.393 ± 0.078 |
| 100 | 1.131 ± 0.500 | 5.598 ± 1.280 |
| 300 | 3.375 ± 0.900 | 25.734 ± 3.668 |

**Table 3.**

| **[**^{**3**}**H]Vinblastine with and without 40 ng/ml Verapamil** | | | | | | |
|---|---|---|---|---|---|---|
| | **(-) Verapamil** | **(-) Verapamil** | **(-) Verapamil** | **(+) Verapamil** | **(+) Verapamil** | **(+) Verapamil** |
| **Time (min)** | **Serosal transfer (ng/mg of protein)** | **Tissue uptake (ng/mg of protein)** | **Total uptake (ng/mg of protein)** | **Serosal transfer (ng/mg of protein)** | **Tissue uptake (ng/mg of protein)** | **Total uptake (ng/mg of protein)** |
| 0 | 0.000± 0.000 | 0.001 ± 0.002 | 0.001 ± 0.000 | 0.001 ± 0.000 | 0.002 ± 0.000 | 0.003 ± 0.000 |
| 30 | 0.002 ± 0.001 | 0.005± 0.000 | 0.007 ± 0.003 | 0.004 ± 0.003 | 0.054 ± 0.002 | 0.058 ± 0.005 |
| 60 | 0.003 ± 0.001 | 0.032 ± 0.005 | 0.035 ± 0.003 | 0.007 ± 0.003 | 0.062 ± 0.019 | 0.069 ± 0.022 |
| 90 | 0.006 ± 0.001 | 0.049 ± 0.006 | 0.055 ± 0.001 | 0.015 ± 0.001 | 0.124 ± 0.009 | 0.139 ± 0.010 |
| 120 | 0.008 ± 0.001 | 0.050± 0.012 | 0.058 ± 0.006 | 0.017 ± 0.006 | 0.125 ± 0.015 | 0.142 ± 0.021 |

**Table 4.**

| **[**^{**14**}**C]Doxorubicin with and without 40 ng/ml Verapamil (n=9 ± SEM)** | | | | | | |
|---|---|---|---|---|---|---|
| | **(-) Verapamil** | **(-) Verapamil** | **(-) Verapamil** | **(+) Verapamil** | **(+) Verapamil** | **(+) Verapamil** |
| **Time (min)** | **Serosal transfer (ng/mg of protein)** | **Tissue uptake (ng/mg of protein)** | **Total uptake (ng/mg of protein)** | **Serosal transfer (ng/mg of protein)** | **Tissue uptake (ng/mg of protein)** | **Total uptake (ng/mg of protein)** |
| 0 | 0.000 ± 0.003 | 0.001 ± 0.003 | 0.001 ± 0.006 | 0.000 ± 0.000 | 0.005 ± 0.002 | 0.005 ± 0.002 |
| 30 | 0.008 ± 0.001 | 0.005 ± 0.006 | 0.013 ± 0.007 | 0.015 ± 0.005 | 0.012 ± 0.004 | 0.027 ± 0.014 |
| 60 | 0.017 ± 0.002 | 0.007 ± 0.005 | 0.024 ± 0.007 | 0.029 ± 0.000 | 0.039 ± 0.013 | 0.068 ± 0.013 |
| 90 | 0.027 ± 0.005 | 0.025 ± 0.001 | 0.052 ± 0.006 | 0.045 ± 0.000 | 0.049 ± 0.020 | 0.094 ± 0.020 |
| 120 | 0.024 ± 0.001 | 0.030 ± 0.003 | 0.054 ± 0.004 | 0.040 ± 0.000 | 0.029 ± 0.004 | 0.069 ± 0.004 |

**Table 5.**

| **[**^{**3**}**H]Vinblastine (1 ng/ml) uptake in rat everted gut sacs (n=6 ± SEM) in the presence of various concentrations of verapamil (60 min)** | | |
|---|---|---|
| **Concn (ng/ml)** | **Serosal transfer (ng/mg of protein)** | **Tissue uptake (ng/mg of protein)** |
| 0 | 0.002 ± 0.000 | 0.032 ±0.005 |
| 4 | 0.005 ± 0.000 | 0.075 ±0.003 |
| 40 | 0.006 ± 0.003 | 0.052 ±0.019 |
| 400 | 0.009 ± 0.001 | 0.092 ±0.008 |
| 4000 | 0.007 ± 0.002 | 0.059 ±0.006 |
| 40000 | 0.004 ± 0.000 | 0.044 ± 0.001 |

**Table 6.**

| **[**^{**14**}**C]Doxorubicin (1 ng/ml) uptake in rat everted gut sacs (n=6 ± SEM) in the presence of various concentrations of verapamil (60 min)** | | |
|---|---|---|
| **Concn (ng/ml)** | **Serosal transfer (ng/mg of protein)** | **Tissue uptake (ng/mg of protein)** |
| 0 | 0.010 ± 0.000 | 0.006 ± 0.000 |
| 4 | 0.029 ± 0.006 | 0.018 ± 0.001 |
| 40 | 0.021 ± 0.005 | 0.039 ±0.015 |
| 400 | 0.015 ± 0.003 | 0.012 ±0.002 |
| 4000 | 0.019 ± 0.003 | 0.013 ± 0.002 |
| 40000 | 0.012 ± 0.003 | 0.007 ± 0.000 |

**Table 7.**

| **[**^{**3**}**H]Vinblastine (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal transfer** | **Incd Ratio Tissue uptake** | **Incd Ratio Total uptake** |
| **Control** | 0.006 ± 0.001 | 0.061 ± 0.006 | 0.067 ± 0.009 | | | |
| **4 ng/ml Verapamil** | 0.006 ± 0.001 | 0.047 ± 0.003 | 0.053 ± 0.009 | 0.9 | 0.8 | 0.8 |
| **40 ng/ml Verapamil** | 0.010 ± 0.001 | 0.124 ± 0.039 | 0.134 ± 0.039 | 1.6 | 2.0 | 2.0 |
| **400 ng/ml Verapamil** | 0.011 ± 0.002 | 0.067 ± 0.016 | 0.078 ± 0.018 | 1.7 | 1.1 | 1.2 |

| | | | | | | |
|---|---|---|---|---|---|---|
| (*) uptake is measured over 90' | | | | | | |

**Table 8.**

| **[**^{**14**}**C]Doxorubicin (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.03 ± 0.00 | 0.02 ± 0.00 | 0.05 ± 0.01 | | | |
| **4 ng/ml Verapamil** | 0.05 ± 0.01 | 0.06 ± 0.02 | 0.11 ± 0.03 | 1.4 | 3.1 | 2.0 |
| **40 ng/ml Verapamil** | 0.05 ± 0.01 | 0.05 ± 0.00 | 0.10 ± 0.00 | 1.0 | 2.7 | 1.9 |
| **400 ng/ml Verapamil** | 0.02 ± 0.01 | 0.02 ± 0.00 | 0.04 ± 0.00 | 0.4 | 1.1 | 0.7 |

| | | | | | | |
|---|---|---|---|---|---|---|
| (*) uptake is measured over 90' | | | | | | |

**Table 9.**

| **[**^{**14**}**C]Doxorubicin (10 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.00 | 0.19 ± 0.03 | 0.24 ± 0.03 | | | |
| **4 ng/ml Verapamil** | 0.13 ± 0.03 | 0.50 ± 0.07 | 0.63 ± 0.07 | 2.8 | 2.6 | 2.6 |
| **40 ng/ml Verapamil** | 0.09 ± 0.01 | 0.27 ± 0.03 | 0.36 ± 0.03 | 1.8 | 1.4 | 1.5 |
| **400 ng/ml Verapamil** | 0.04 ± 0.00 | 0.12 ± 0.01 | 0.16 ± 0.01 | 0.9 | 0.6 | 0.7 |

| | | | | | | |
|---|---|---|---|---|---|---|
| (*) uptake is measured over 90' | | | | | | |

**Table 10.**

| **[**^{**14**}**C]Doxorubicin (100 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.07 | 0.75 ± 0.13 | 1.25 ± 0.07 | | | |
| **4 ng/ml Verapamil** | 0.59 ± 0.11 | 0.86 ± 0.11 | 1.45 ± 0.08 | 1.2 | 1.1 | 1.2 |

| | | | | | | |
|---|---|---|---|---|---|---|
| (*) uptake is measured over 90' | | | | | | |

### Example 2. In vitro transfer of [⁵H]Vinblastine and [¹⁴C]Doxorubicin in the presence of alginates.

Ascophyllum and flavicam were incubated at two concentrations (0.5 and 1 mg/ml) with 1ng/ml [³H]vinblastine, and 0.5 mg/ml of the alginates were incubated with 1 ng/ml [¹⁴C]doxorubicin, 1 ml, containing the desired concentration of alginate plus either radiolabelled anticancer agent, was added to 9 ml of oxygenated tissue culture medium 199. The flasks were stopped using gas-tight silicone bungs. Sacs were incubated at 37°C in a Grant Instruments (Cambridge, U.K., 5540-5) shaking water bath (70 beats/min). At the desired time points, sacs were removed, washed three times in saline and blotted dry. Sacs were then weighed and the serosal contents (1 ml) drained into small tubes. Sacs were re-weighed after draining to calculate accurately the volume inside each sac. The sacs were then digested individually in 5 ml of 5 M NaOH at 37°C overnight, and then samples of the tissue digest (0.8 ml) were neutralised with 5 M HCl (0.2 ml). The protein content of the digest was determined by the Lowry method as modified by Peterson (Peterson GL (1983) Determination of Total Protein. Methods in Enzymology. 91: 95-119).
To estimate radioactivity, scintillation fluid (4 ml) was added to samples of the incubation media (2 x 1 ml), the serosal fluid (1 ml) and tissue digest (1 ml), was added and samples were counted using a Beckham scintillation counter. The drug present in tissue was expressed as ng /mg of total sac protein and the serosal transport as ng transferred /mg of total sac protein.
Gut sac viability: In order to verify the integrity of the sacs, glucose was measured both in the incubation medium and in the sac contents using a modification of the method by Dahlqvist (Dahlqvist A (1968) Assay of intestinal disaccharidases. Anal. Biochem. 22: 99-107). Briefly, 20 µl of sample were incubated for 45 minutes with 1 ml of glucose oxidase reagent (0.2% Triton-X100 (w/v in ethanol)), 10µg/ml O-dianisidine-HCl, 1 µg/ml peroxidase, 200 µg/ml glucose oxidase in 0.5 M Tris/HCl, pH 7.2). The reaction was stopped by the addition of 2 ml of 5 M HCl, and the absorbance measured at 525 nm.
**Results:-** Tissue viability was compromised at 1 mg/ml ascophyllum and flavicam in conjunction with 1 ng/ml [³H]vinblastine. Thus only 0.5 mg/ml concentrations were tested on 1 ng/ml [¹⁴C]doxorubicin. Ascophyllum at 0.5 mg/ml increased [³H]vinblastine accumulation into the tissue xl.3 and across to the serosa by x2.0; having a total ratio of increase of x1.4. 0.5 mg/ml flavicam did not show any increase on the uptake of [³H]vinblastine. With respect to [¹⁴C]doxorubicin, 0.5 mg/ml ascophyllum increased [¹⁴C]doxorubicin tissue accumulation by x2.4, had no increase on the drug's serosal transfer, having an overall increase of x1.4 over drug alone. 0.5 mg/ml flavicam (Figure 13) increased [¹⁴C]doxorubicin tissue accumulation by x3.6 and aided its transfer to the serosal side by x3.6; having an overall increase of x2.6. A summary of these data can be seen in Table 11 for [³H]vinblastine studies and Tables 12 and 13 for [¹⁴C]doxorubicin.

**Table 11.**

| **[**^{**3**}**H]Vinblastine (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal transfer** | **Incd Ratio Tissue uptake** | **Incd Ratio Total uptake** |
| **Control** | 0.006 ± 0.001 | 0.061 ± 0.006 | 0.067 ± 0.009 | | | |
| **0.5 mg/ml Ascophyllum** | 0.013 ± 0.003 | 0.079 ± 0.010 | 0.092 ± 0.012 | 2.0 | 1.3 | 1.4 |
| **1 mg/ml Ascophyllum** | 0.008 ± 0.001 | 0.054 ± 0.011 | 0.062 ± 0.012 | 1.3 | 0.9 | 0.9 |
| **0.5 mg/ml Flavicam** | 0.007 ± 0.001 | 0.059 ± 0.008 | 0.066 ± 0.009 | 1.1 | 1.0 | 1.0 |
| **1 mg/ml Flavicam** | 0.007 ± 0.001 | 0.030 ± 0.010 | 0.037 ± 0.010 | 1.1 | 0.5 | 0.6 |

**Table 12.**

| **[**^{**14**}**C]Doxorubicin (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.03 ± 0.00 | 0.02 ± 0.00 | 0.05 ± 0.01 | | | |
| **0.5 mg/ml Ascophyllum** | 0.03 ± 0.01 | 0.05 ± 0.01 | 0.08 ± 0.02 | 0.9 | 2.4 | 1.5 |
| **0.5 mg/ml Flavicam** | 0.07 ± 0.02 | 0.07 ± 0.00 | 0.14 ± 0.01 | 2.1 | 3.6 | 2.6 |

| | | | | | | |
|---|---|---|---|---|---|---|
| (*) uptake is measured over 90' | | | | | | |

**Table 13.**

| **[**^{**14**}**C]Doxorubicin (10 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.00 | 0.19 ± 0.03 | 0.24 ± 0.03 | | | |
| **0.5 mg/ml Ascophyllum** | 0.07 ± 0.01 | 0.23 ± 0.02 | 0.30 ± 0.02 | 1.4 | 1.2 | 1.2 |
| **1 mg/ml Flavicam** | 0.08 ± 0.01 | 0.24 ± 0.04 | 0.33 ± 0.04 | 1.8 | 1.2 | 1.4 |
| **0.5 mg/ml Flavicam** | 0.08 ± 0.00 | 0.23 ± 0.2 | 0.31 ± 0.02 | 1.7 | 1.2 | 1.3 |

| | | | | | | |
|---|---|---|---|---|---|---|
| (*) uptake is measured over 90' | | | | | | |

### Example 3. In vitro transfer of [³H]Vinblastine and [¹⁴C]Doxorubicin in the presence of gums.

Xanthan gum was incubated at a concentration of 0.5 mg/ml with either vinblastine or doxorubicin with rat everted gut sacs. Gellan gum was incubated at 0.5 and 1 mg/ml in the case of [¹⁴C]doxorubicin studies and at 0.5 mg/ml in the case of [3H]vinblastine studies, with rat everted gut sacs. Both anticancer agents were at 1 ng/ml. 1 ml of the desired concentration of polymers was added to 9 ml of oxygenated media 199 containing 1 ng/ml [¹⁴C]doxorubicin or [³H]vinblastine. The flasks were stopped using gas-tight silicone bungs. Sacs were incubated at 37°C in a Grant Instruments (Cambridge, U.K., 5540-5) shaking water bath (70 beats/min). At the desired time points, sacs were removed, washed three times in saline and blotted dry. Sacs were then weighed and the serosal contents (1 ml) drained into small tubes. Sacs were re-weighed after draining to calculate accurately the volume inside each sac. The sacs were then digested individually in 5 ml of 5 M NaOH at 37°C overnight, and then samples of the tissue digest (0.8 ml) were neutralised with 5 M HCl (0.2 ml). The protein content of the digest was determined by the Lowry method as modified by Peterson (Peterson GL (1983) Determination of Total Protein. Methods in Enzymology. 91: 95-119).
To estimate radioactivity, scintillation fluid (4 ml) was added to samples of the incubation media (2 x 1 ml), the serosal fluid (1 ml) and tissue digest (1 ml), was added and samples were counted using a Beckham scintillation counter. The drug present in tissue was expressed as ng/mg of total sac protein and the serosal transport as ng transferred /mg of total sac protein.
Gut sac viability: In order to verify the integrity of the sacs, glucose was measured both in the incubation medium and in the sac contents using a modification of the method by Dahlqvist (Dahlqvist A (1968) Assay of intestinal disaccharidases. Anal. Biochem. 22: 99-107). Briefly, 20 µl of sample were incubated for 45 minutes with 1 ml of glucose oxidase reagent (0.2% Triton-X100 (w/v in ethanol)), 10µg/ml O-dianisidine-HCl, 1 µg/ml peroxidase, 200 µg/ml glucose oxidase in 0.5 M Tris/HCl, pH 7.2). The reaction was stopped by the addition of 2 ml of 5 M HCl, and the absorbance measured at 525 nm.
**Results:-** Sac viablility was maintained in the presence of both gums. Xanthan gum increased [³H]vinblastine tissue accumulation by x1.5 and its serosal transfer was increased by x2.8, having an overall increase of x1.6. [¹⁴C]Doxorubicin tissue accumulation was increased by x2.2 whereas serosal transfer was not affected.
Gellan gum increased [³H]vinblastine serosal tranfer but tissue levels were the same as the control. [¹⁴C]Doxorubicin uptake was not affected by 1mg/ml Gellan gum but at 0.5 mg/ml Gellan gum, there was a x3.0 increase in tissue levels and x2.4 increase in serosal tranfer, having an overall increase of x2.0. A summary of these data can be seen in Table 14 for [³H]vinblastine studies and Tables 15-17 for [¹⁴C]doxorubicin.

**Table 14.**

| **[**^{**3**}**H]Vinblastine (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal transfer** | **Incd Ratio Tissue uptake** | **Incd Ratio Total uptake** |
| **Control** | 0.006 ± 0.001 | 0.061 ± 0.006 | 0.067 ± 0.009 | | | |
| **0.5 mg/ml Xanthan gum** | 0.018 ± 0.003 | 0.089 ± 0.015 | 0.107 ± 0.017 | 2.8 | 1.5 | 1.6 |
| **0.5 mg/ml Gellan gum** | 0.009 ± 0.001 | 0.050 ± 0.005 | 0.059 ± 0.006 | 1.4 | 0.8 | 0.9 |

| | | | | | | |
|---|---|---|---|---|---|---|
| (*) uptake is measured over 90' | | | | | | |

**Table 15.**

| **[**^{**14**}**C]Doxorubicin (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.03 ± 0.00 | 0.02 ± 0.00 | 0.05 ± 0.01 | | | |
| **0.5 mg/ml Gellan gum** | 0.04 ± 0.00 | 0.06 ± 0.01 | 0.10 ± 0.01 | 2.4 | 3.0 | 1.9 |
| **1 mg/ml Gellan gum** | 0.03 ± 0.01 | 0.03 ± 0.00 | 0.06 ± 0.00 | 0.8 | 1.3 | 1.1 |
| **0.5 mg/ml Xanthan gum** | 0.03 ± 0.01 | 0.04 ± 0.01 | 0.08 ± 0.01 | 1.1 | 2.2 | 1.5 |

**Table 16.**

| **[**^{**14**}**C]Doxorubicin (10 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.00 | 0.19 ± 0.03 | 0.24 ± 0.03 | | | |
| **0.5 mg/ml Xanthan gum** | 0.08 ± 0.01 | 0.24 ± 0.05 | 0.32 ± 0.04 | 1.8 | 1.2 | 1.3 |
| **0.5 mg/ml Gellan gum** | 0.07 ± 0.01 | 0.16 ± 0.02 | 0.23 ± 0.03 | 1.5 | 0.8 | 0.9 |

| | | | | | | |
|---|---|---|---|---|---|---|
| (*) uptake is measured over 90' | | | | | | |

**Table 17.**

| **[**^{**14**}**C]Doxorubicin (100 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.07 | 0.75 ± 0.13 | 1.25 ± 0.07 | | | |
| **0.5 mg/ml Xanthan gum** | 1.18 ± 0.14 | 2.17 ± 0.23 | 3.35 ± 0.09 | 2.3 | 2.9 | 2.7 |
| **0.5 mg/ml Gellan gum** | 1.35 ± 0.27 | 1.81 ± 0.34 | 3.16 ± 0.31 | 2.7 | 2.4 | 2.5 |

| | | | | | | |
|---|---|---|---|---|---|---|
| (*) uptake is measured over 90' | | | | | | |

### Example 4. In vitro transfer of [³H]Vinblastine and [¹⁴C]Doxorubicin in the presence of polidocanol.

1ml of 0.5 mg/ml polidocanol (MWt 583) was added to 9 ml of oxygenated media 199 containing 1 ng/ml [³H]vinblastine. In [¹⁴C]doxorubicin studies 1 ml of 0.25 mg/ml polidocanol was added to 9 ml of oxygenated 199 media containing 10 ng/ml [¹⁴C]doxorubicin. The flasks were stopped using gas-tight silicone bungs. Sacs were incubated at 37°C in a Grant Instruments (Cambridge, U.K., 5540-5) shaking water bath (70 beats/min). At the desired time points, sacs were removed, washed three times in saline and blotted dry. Sacs were then weighed and the serosal contents (1 ml) drained into small tubes. Sacs were re-weighed after draining to calculate accurately the volume inside each sac. The sacs were then digested individually in 5 ml of 5 M NaOH at 37°C overnight, and then samples of the tissue digest (0.8 ml) were neutralised with 5 M HCl (0.2 ml). The protein content of the digest was determined by the Lowry method as modified by Peterson (Peterson GL (1983) Determination of Total Protein. Methods in Enzymology. 91: 95-119).
To estimate radioactivity, scintillation fluid (4 ml) was added to samples of the incubation media (2 x 1 ml), the serosal fluid (1 ml) and tissue digest (1 ml), was added and samples were counted using a Beckham scintillation counter. The drug present in tissue was expressed as ng /mg of total sac protein and the serosal transport as ng transferred /mg of total sac protein.

Gut sac viability: In order to verify the integrity of the sacs, glucose was measured both in the incubation medium and in the sac contents using a modification of the method by Dahlqvist (Dahlqvist A (1968) Assay of intestinal disaccharidases. Anal. Biochem. 22: 99-107). Briefly, 20 µl of sample were incubated for 45 minutes with 1 ml of glucose oxidase reagent (0.2% Triton-X100 (w/v in ethanol)), 10µg/ml O-dianisidine-HCl, 1 µg/ml peroxidase, 200 µg/ml glucose oxidase in 0.5 M Tris/HCl, pH 7.2). The reaction was stopped by the addition of 2 ml of 5 M HCl, and the absorbance measured at 525 nm.

**Results:-** Gut sac viability was not compromised in the presence of polidocanol within the concentrations tested. [³H]Vinblastine tissue uptake was improved by x1.2, serosal tranfer by x4.5, having an overall increase of drug uptake of x1.5. [¹⁴C]Doxorubicin displayed a different pattern where tissue accumulation was higher (x2.7) than serosal transfer (x1.7), having a total increase of x2.1. A summary of these data can be seen in Table 18 for [³H]vinblastine studies and Table 19 for [¹⁴C]doxorubicin.

**Table 18.**

| **[**^{**3**}**H]Vinblastine (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal transfer** | **Incd Ratio Tissue uptake** | **Incd Ratio Total uptake** |
| **Control** | 0.006 ± 0.001 | 0.061 ± 0.006 | 0.067 ± 0.009 | | | |
| **0.5 mg/ml polidocanol** | 0.029 ± 0.003 | 0.073 ± 0.009 | 0.102 ± 0.012 | 4.5 | 1.2 | 1.5 |

| | | | | | | |
|---|---|---|---|---|---|---|
| (*) uptake is measured over 90' | | | | | | |

**Table 19.**

| **[**^{**14**}**C]Doxorubicin (10 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.00 | 0.19 ± 0.03 | 0.24 ± 0.03 | | | |
| **0.25 mg/ml Polidocanol** | 0.08 ± 0.01 | 0.53 ± 0.14 | 0.51 ± 0.13 | 1.7 | 2.7 | 2.1 |

| | | | | | | |
|---|---|---|---|---|---|---|
| (*) uptake is measured over 90' | | | | | | |

### Example 5. In vitro transfer of [³H]Vinblastine and [¹⁴C]Doxorubicin in the presence of polysaccharides.

1ml of 0.5 mg/ml or 1 mg/ml Dextran was added to 9 ml of oxygenated media 199 containing 1 ng/ml [³H]vinblastine or [¹⁴C]doxorubicin. Moreover, 1 ml of 1 mg/ml was added to 9 ml of oxygenated 199 media containing 10 ng/ml [¹⁴C]doxorubicin. In fucoidan stidies, 0.5 and 1mg/ml were used to increase [³H]vinblastine uptake and 1 mg/ml was used for [¹⁴C]doxorubicin studies. The flasks were stopped using gas-tight silicone bungs. Sacs were incubated at 37°C in a Grant Instruments (Cambridge, U.K., 5540-5) shaking water bath (70 beats/min). At the desired time points, sacs were removed, washed three times in saline and blotted dry. Sacs were then weighed and the serosal contents (1 ml) drained into small tubes. Sacs were re-weighed after draining to calculate accurately the volume inside each sac. The sacs were then digested individually in 5 ml of 5 M NaOH at 37°C overnight, and then samples of the tissue digest (0.8 ml) were neutralised with 5 M HCl (0.2 ml). The protein content of the digest was determined by the Lowry method as modified by Peterson (Peterson GL (1983) Determination of Total Protein. Methods in Enzymology. 91: 95-119).

To estimate radioactivity, scintillation fluid (4 ml) was added to samples of the incubation media (2 x 1 ml), the serosal fluid (1 ml) and tissue digest (1 ml), was added and samples were counted using a Beckham scintillation counter. The drug present in tissue was expressed as ng /mg of total sac protein and the serosal transport as ng transferred /mg of total sac protein.

Gut sac viability: In order to verify the integrity of the sacs, glucose was measured both in the incubation medium and in the sac contents using a modification of the method by Dahlqvist (Dahlqvist A (1968) Assay of intestinal disaccharidases. Anal. Biochem. 22: 99-107). Briefly, 20 µ1 of sample were incubated for 45 minutes with 1 ml of glucose oxidase reagent (0.2% Triton-X100 (w/v in ethanol)), 10µg/ml O-dianisidine-HCI, 1 µg/ml peroxidase, 200 µg/ml glucose oxidase in 0.5 M Tris/HCl, pH 7.2). The reaction was stopped by the addition of 2 ml of 5 M HCl, and the absorbance measured at 525 nm.

**Results:-** Gut sac viability was not compromised in the presence of either concentration of dextran or fucoidan.
0.5 mg/ml Dextran had no effect on [³H]vinblastine uptake. On 1 ng/ml doxorubicin, the presence of 0.5 mg/ml dextran did not aid the drug's uptake into the tissue, but increased the drug's serosal transfer by x2.3, having an overall increase of x1.6.
At 1 mg/ml Dextran [³H]vinblastine tissue uptake was improved by x1.3, serosal tranfer by xl.9, having an overall increase of drug uptake of x1.3. [¹⁴C]doxorubicin displayed a different pattern where tissue accumulation was higer (x2.9) than serosal transfer (x0.9), having a total increase of x1.8.
At a 10 ng/ml [¹⁴C]Doxorubicin concentration, the presence of 1mg/ml Dextran, increased uptake into the tissue by x1.8, transfer to the serosa by x2.7, having a total increase of x2.0.
Fucoidan, at either concentration tested, did not have an effect on [³H]vinblastine uptake. In contrast, 1 mg/ml fucoidan aided [¹⁴C]doxorubicin tissue accumulation by x2.8 but no serosal increase was observed, having an overall increase of x1.8. A summary of these data can be seen in Table 20 for [³H]vinblastine studies and Tables 21-23 for [¹⁴C]doxorubicin.

**Table 20.**

| **[**^{**3**}**H]Vinblastine (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal transfer** | **Incd Ratio Tissue uptake** | **Incd Ratio Total uptake** |
| **Control** | 0.006 + 0.001 | 0.061 + 0.006 | 0.067 ± 0.009 | | | |
| **0.5 mg/ml Fucoidin** | 0.0076± 0.001 | 0.035 ± 0.008 | 0.041 ± 0.008 | 0.9 | 0.6 | 0.6 |
| **1 mg/ml Fucoidin** | 0.0076± 0.001 | 0.059 ± 0.010 | 0.065 ± 0.010 | 1.0 | 1.0 | 1.0 |
| **0.5 mg/ml Dextran** | 0.004 ± 0.002 | 0.033 ± 0.002 | 0.037 ± 0.003 | 0.7 | 0.5 | 0.6 |
| **1 mg/ml Dextran** | 0.012 ± 0.002 | 0.076 ± 0.007 | 0.088 ± 0.007 | 1.9 | 1.3 | 1.3 |

| | | | | | | |
|---|---|---|---|---|---|---|
| (*) uptake is measured over 90' | | | | | | |

**Table 21.**

| **[**^{**14**}**C]Doxorubicin (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.03 ± 0.00 | 0.02 ± 0.00 | 0.05 ± 0.01 | | | |
| **0.5 mg/ml Dextran** | 0.03 ± 0.00 | 0.04 ± 0.01 | 0.08 ± 0.01 | 1.1 | 2.3 | 1.6 |
| **1 mg/ml Dextran** | 0.04 ± 0.01 | 0.06 ± 0.01 | 0.09 ± 0.02 | 0.9 | 2.9 | 1.8 |
| **1 mg/ml Fucoidin** | 0.03 ± 0.01 | 0.05 ± 0.02 | 0.09 ± 0.02 | 0.5 | 2.8 | 1.8 |

**Table 22.**

| **[**^{**14**}**C]Doxorubicin (10 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.00 | 0.19 ± 0.03 | 0.24 ± 0.03 | | | |
| **0.5 mg/ml Dextran** | 0.13 ± 0.03 | 0.36 ± 0.03 | 0.48 ± 0.05 | 2.7 | 1.8 | 2.0 |
| **1 mg/ml Fucoidin** | 0.03 ± 0.00 | 0.06 ± 0.01 | 0.09 ± 0.01 | 0.7 | 0.3 | 0.4 |

**Table 23.**

| **[**^{**14**}**C]Doxorubicin (100 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.07 | 0.75 ± 0.13 | 1.25 ± 0.07 | | | |
| **1 mg/ml Dextran** | 0.59 ± 0.02 | 0.74 ± 0.26 | 1.33 ± 0.26 | 1.2 | 1.0 | 1.1 |

| | | | | | | |
|---|---|---|---|---|---|---|
| (*) uptake is measured over 90' | | | | | | |

### Example 6. In vitro transfer of [³H]Vinblastine and [¹⁴C]Doxorubicin in the presence of dendrimers.

1 ml containing of 0.5 mg/ml G3 dendrimer was added to 9 ml of oxygenated media 199 containing 1 ng/ml [³H]vinblastine. 1 ml of 50 µg/ml G3 dendrimer was added to 9 ml of oxygenated media 199 containing 10 ng/ml [¹⁴C]doxorubicin. 1 ml containing of 0.5 mg/ml G3.5 dendrimer was added to 9 ml of oxygenated media 199 containing either 1 ng/ml [³H]vinblastine or 1 ng/ml [¹⁴C]doxorubicin. Also, 1 ml containing of 50 µg/ml G3.5 dendrimer was added to 9 ml of oxygenated media 199 containing either 10 ng/ml [¹⁴C]doxorubicin. The flasks were stopped using gas-tight silicone bungs. Sacs were incubated at 37°C in a Grant Instruments (Cambridge, U.K., 5540-5) shaking water bath (70 beats/min). At the desired time points, sacs were removed, washed three times in saline and blotted dry. Sacs were then weighed and the serosal contents (1 ml) drained into small tubes. Sacs were re-weighed after draining to calculate accurately the volume inside each sac. The sacs were then digested individually in 5 ml of 5 M NaOH at 37°C overnight, and then samples of the tissue digest (0.8 ml) were neutralised with 5 M HCl (0.2 ml). The protein content of the digest was determined by the Lowry method as modified by Peterson (Peterson GL (1983) Determination of Total Protein. Methods in Enzymology. 91: 95-119).

To estimate radioactivity, scintillation fluid (4 ml) was added to samples of the incubation media (2 x 1 ml), the serosal fluid (1 ml) and tissue digest (1 ml), was added and samples were counted using a Beckham scintillation counter. The drug present in tissue was expressed as ng /mg of total sac protein and the serosal transport as ng transferred /mg of total sac protein.

Gut sac viability: In order to verify the integrity of the sacs, glucose was measured both in the incubation medium and in the sac contents using a modification of the method by Dahlqvist (Dahlqvist A (1968) Assay of intestinal disaccharidases. Anal. Biochem. 22: 99-107). Briefly, 20 µl of sample were incubated for 45 minutes with 1 ml of glucose oxidase reagent (0.2% Triton-X100 (w/v in ethanol)), 10µg/ml O-dianisidine-HCl, 1 µg/ml peroxidase, 200 µg/ml glucose oxidase in 0.5 M Tris/HCl, pH 7.2). The reaction was stopped by the addition of 2 ml of 5 M HCl, and the absorbance measured at 525 nm.

**Results:-** Sac viablility was maintained in the presence of both dendrimers. G3 increased by x3.1 [³H]vinblastine tissue accumulation and its serosal transfer was increased by x1.2, having an overall increase of x3.0. In the presence of 10 ng/ml [¹⁴C]Doxorubicin, G3 increased tissue accumulation by x4.1 and serosal tranfer by x1.8, having an overall increase of x3.7.

G3.5 increased [³H]vinblastine serosal tranfer by x1.6 but tissue levels were the same as the control. 1 ng/ml [¹⁴C]Doxorubicin accumulation into tissue was increased in the presence of 0.5mg/ml G3.5 by x2.4, whereas serosal transfer was not affected, having an overall increase of x1.5. When 50 µg/ml G3.5 was incubated with 10 ng/ml [¹⁴C]Doxorubicin, increased the drug's tissue uptake by x1.6, serosal transfer by 1.2 and total uptake by x1.5. A summary of these data can be seen in Table 24 for [³H]vinblastine studies and Tables 25-26 for [¹⁴C]doxorubicin.

**Table 24.**

| **[**^{**3**}**H]Vinblastine (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal transfer** | **Incd Ratio Tissue uptake** | **Incd Ratio Total uptake** |
| **Control** | 0.006 ± 0.001 | 0.061 ± 0.006 | 0.067 ± 0.009 | | | |
| **0.5 mg/ml G3.5** | 0.010 ± 0.002 | 0.058 ± 0.008 | 0.068 ± 0.010 | 1.6 | 1.0 | 1.0 |
| **0.5 mg/ml G 3** | 0.008 ± 0.001 | 0.190 ± 0.014 | 0.198 ± 0.015 | 1.2 | 3.1 | 3.0 |

**Table 25.**

| **[**^{**14**}**C]Doxorubicin (1 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.03 ± 0.00 | 0.02 ± 0.00 | 0.05 ± 0.01 | | | |
| **0.5 mg/ml G 3.5** | 0.03 ± 0.01 | 0.05 ± 0.02 | 0.08 ± 0.01 | 1.1 | 2.4 | 1.5 |

| | | | | | | |
|---|---|---|---|---|---|---|
| (*) uptake is measured over 90' | | | | | | |

**Table 26.**

| **[**^{**14**}**C]Doxorubicin (10 ng/ml) uptake (*) in rat everted gut sacs (n=9 ± SEM)** | | | | | | |
|---|---|---|---|---|---|---|
| | **Serosal transfer (ng/mg of protein)** | **Tissue uptake (ng/mg of protein)** | **Total uptake (ng/mg of protein)** | **Incd Ratio Serosal tranfer** | **Incd Ratio Tissue uptake** | **Incd Ratio Total uptake** |
| **Control** | 0.05 ± 0.00 | 0.19 ± 0.03 | 0.24 ± 0.03 | | | |
| **0.5 mg/ml G3.5** | 0.05 ± 0.00 | 0.30 ± 0.04 | 0.36 ± 0.04 | 1.2 | 1.6 | 1.5 |
| **0.5 mg/ml G3** | 0.08 ± 0.01 | 0.8 ± 0.06 | 0.89 ± 0.07 | 1.8 | 4.1 | 3.7 |

| | | | | | | |
|---|---|---|---|---|---|---|
| (*) uptake is measured over 90' | | | | | | |

### Example 7. In vivo biodistribution of [³H]vinblastine after oral gavage (100 ng/kg)

Male Wistar rats (n=3) (average weight 200-300 g; 11-12 weeks old) were given a single dose of [³H]vinblastine in 0.4 ml (100 ng/kg, ~1.7 µCi) by oral gavage. Animals were allowed free access to water throughout, but starved overnight before dosing. Food was made available 2 hours later. The rats were kept in metabolic cages to facilitate collection of urine and faeces for measurement of radioactivity and to prevent coprophagia. Animals were sacrificed at different time points (5 and 30 minutes, 1, 5 and 24 hours) using CO₂ and all systemic organs were removed. The GI tract was also excised and separated into stomach, small intestine, cecum and large intestine. Washings of each section were also collected. Blood was obtained by cardiac puncture. Subsequently, organs were homogenised (using a blade homogeniser) and aliquots were taken, and mixed with scintillation liquid. Radioactivity was assessed using a β-counter. To compensate for quenching, all samples after the initial reading were spiked with a known amount of radioactivity and subsequently re-read. The blood volume of the rat was calculated assuming 7.2ml blood/100g rat.

**Results:-** [³H]Vinblastine biodistribution is shown in Table 27. It can be seen that the drug did not seem to accumulate in any major organs and its levels in blood remained constant throughout the time course study.Approximately 1.2-1.6% of the dose can be seen in the stomach, after 1 hour (h). Subsequent time points suggest that [³H]vinblastine moves down the GI tract after 1 hour, concentrating in the small intestinal (SI) wash, and almost no absorption takes place in the SI. After 5 hours, 1.7±0.3% is found in the cecum and 0.33±0.03% is found in the faeces. After 24 hours, 3% is in the blood and 0.7% in the heart; all other organs do not show any significant levels of the drug. Table 28 summarises the AUC values in all major organs.

**Table 27.**

| **[**^{**3**}**H]Vinblastine (100 ng/kg) organ biodistribution (% Dose/organ weight)** | | | | | |
|---|---|---|---|---|---|
| | **Time= 5 min** | **Time= 30 min** | **Time= 1 h** | **Time= 5 h** | **Time= 24 h** |
| **Blood** | 2.87 ± 1.32 | 4.25 ± 1.46 | 2.00 ± 0.42 | 1.32 ± 0.58 | 3.30 ± 1.2 |
| **Heart** | 0.13 ± 0.02 | 0.09 ± 0.03 | 0.11 ± 0.01 | 0.07 ± 0.03 | 0.7 ± 0.1 |
| **Kidney** | 0.04 ± 0.01 | 0.03 ± 0.01 | 0.06 ± 0.02 | 0.05 ± 0.01 | 0.03 ± 0.00 |
| **Lung** | 0.06 ± 0.01 | 0.05 ± 0.01 | 0.08 ± 0.02 | 0.06 ± 0.01 | 0.06 ± 0.01 |
| **Liver** | 0.04 ± 0.01 | 0.04 ± 0.01 | 0.05 ± 0.01 | 0.34 ± 0.06 | 0.03 ± 0.01 |
| **Spleen** | 0.09 ± 0.02 | 0.09 ± 0.03 | 0.11 ± 0.02 | 0.13 ± 0.06 | 0.11 ± 0.01 |
| **Small Intestine** | 0.08 ± 0.02 | 0.17 ± 0.03 | 0.18 ± 0.06 | 0.08 ± 0.02 | 0.03 ± 0.01 |
| **Small Intestinal Wash** | 0.57 ± 0.08 | 3.25 ± 0.3 | 5.35 ± 1.90 | 1.96 ± 1.18 | 0.06 ± 0.01 |
| **Large Intestine** | 0.03 ± 0.01 | 0.03 ± 0.01 | 0.07 ± 0.04 | 0.06 ± 0.03 | 0.03 ± 0.01 |
| **Large Intestinal Wash** | 0.05 ± 0.01 | 0.07 ± 0.01 | 0.018 ± 0.06 | 0.45 ± 0.36 | 0.09 ± 0.01 |
| **Cecum** | 0.03 ± 0.01 | 0.04 ± 0.01 | 0.05 ± 0.01 | 1.72 ± 0.43 | 0.03 ± 0.01 |
| **Stomach** | 1.23 ± 0.38 | 1.42 ± 0.08 | 1.65 ± 0.27 | 0.05 ± 0.00 | 0.02 ± 0.01 |
| **Urine** | nc | nc | 0.11 ± 0.01 | 0.04 ± 0.00 | 0.11 ± 0.02 |
| **Faeces** | nc | nc | nc | 0.33 ± 0.03 | 0.09 ± 0.00 |

**Table 28.**

| **Area Under the Curve (% Dose/organ weight, 5min-24 h)of [**^{**3**}**H]Vinblastine (100 ng/kg) (n=3 ± SEM) in control** | |
|---|---|
| **Organ** | **AUC Control** |
| Blood | 53.47 |
| Heart | 1.80 |
| Kidney | 0.98 |
| Lung | 1.54 |
| Liver | 4.36 |
| Spleen | 2.78 |
| Small Intestine | 1.69 |
| Small Intestinal Wash | 36.66 |
| Large Intestine | 1.08 |
| Large Intestinal Wash | 6.46 |
| Cecum | 20.20 |
| Stomach | 5.34 |
| Urine | 1.79 |
| Faeces | 4.58 |

### Example 8. In vivo biodistribution of [³H]vinblastine (100 ng/kg) in rats when co-administered with ascophyllum (250 mg/kg)

**Methods:-** Male Wistar rats (n=3) (average weight 200-300 g; 11-12 weeks old) were given a single dose of [³H]vinblastine in 3 ml (100 ng/kg, ~1.7 µCi) with xanthan gum (250 mg/kg)by oral gavage. Animals were allowed free access to water throughout, but starved overnight before dosing. Food was made available 2 hours later. The rats were kept in metabolic cages to facilitate collection of urine and faeces for measurement of radioactivity and to prevent coprophagia. Animals were sacrificed at different time points (5 and 30 minutes, 1, 5 and 24 hours) using CO₂ and all systemic organs were removed. The GI tract was also excised and separated into stomach, small intestine, cecum and large intestine.

Washings of each section were collected too. Blood was obtained by cardiac puncture. Subsequently, organs were homogenised (using a blade homogeniser) and aliquots were taken, and mixed with scintillation liquid. Radioactivity was assessed using a ß-counter. To compensate for quenching, all samples after the initial reading were spiked with a known amount of radioactivity and subsequently re-read. The blood volume of the rat was calculated assuming 7.2ml blood/100g rat.

**Results:-** [³H]Vinblastine biodistribution in the presence of ascophyllum is shown in Table 29. In the presence of ascophyllum there is a [³H]Vinblastine increase in AUC of x 1.7 in blood, x3.3 in large intestine and x1.8 in urine. AUC values are shown in Table 30.

**Table 29.**

| **[**^{**3**}**H]Vinblastine (100 ng/kg) organ biodistribution (% Dose/organ weight) in the presence of 250 mg/kg Ascophyllum (n=3 ± SEM)** | | | | | |
|---|---|---|---|---|---|
| | **Time= 5 min** | **Time= 30 min** | **Time= 1 h** | **Time= 5 h** | **Time= 24 h** |
| **Blood** | 12.15 ± 0.40 | 0.63 ± 0.09 | 1.01 ± 0.26 | 3.56 ± 0.22 | 4.87 ± 2.56 |
| **Heart** | 0.27 ± 0.05 | 0.07 ± 0.01 | 0.04 ± 0.00 | 0.14 ± 0.08 | 0.6 ± 0.00 |
| **Kidney** | 0.09 ± 0.01 | 0.03 ± 0.00 | 0.02 ± 0.00 | 0.04 ± 0.00 | 0.04 ± 0.01 |
| **Lung** | 0.16 ± 0.02 | 0.05 ± 0.00 | 0.09 ± 0.02 | 0.07 ± 0.01 | 0.05 ± 0.01 |
| **Liver** | 0.17 ± 0.03 | 0.04 ± 0.00 | 0.05 ± 0.00 | 0.05 ± 0.00 | 0.04 ± 0.01 |
| **Spleen** | 0.19 ± 0.04 | 0.10 ± 0.01 | 0.09 ± 0.02 | 0.11 ± 0.01 | 0.11 ± 0.02 |
| **Small Intestine** | 0.44 ± 0.06 | 0.13 ± 0.03 | 0.14 ± 0.02 | 0.03 ± 0.01 | 0.01 ± 0.00 |
| **Small Intestinal Wash** | 2.89 ± 0.22 | 2.14 ± 0.29 | 2.60 ± 0.37 | 0.54 ± 0.21 | 0.11 ± 0.03 |
| **Large Intestine** | 0.07 ± 0.02 | 0.02 ± 0.00 | 0.02 ± 0.00 | 0.07 ± 0.02 | 0.02 ± 0.01 |
| **Large Intestinal Wash** | 0.09 ± 0.02 | 0.03 ± 0.01 | 0.04 ± 0.01 | 1.71 ± 0.16 | 0.15 ± 0.05 |
| **Cecum** | 0.06 ± 0.03 | 0.02 ± 0.00 | 0.02 ± 0.00 | 1.03 ± 0.30 | 0.09 ± 0.06 |
| **Stomach** | 0.43 ± 0.08 | 0.47 ± 0.06 | 0.17 ± 0.05 | 0.08 ± 0.04 | 0.02 ± 0.02 |
| **Urine** | nc | nc | nc | 0.17 ± 0.05 | 0.14 ± 0.06 |
| **Faeces** | nc | nc | nc | nc | 0.10 ± 0.04 |

**Table 30.**

| **Area Under the Curve (% Dose/organ weight, 5min-24 h)of [**^{**3**}**H]Vinblastine (100 ng/kg) (n=3 ± SEM) in control and in presence of ascophyllum** | | | |
|---|---|---|---|
| **Organ** | **AUC Control** | **AUC (+) 250 mg/kg Ascophyllum** | **Ratio Ascophyllum/ Control** |
| **Blood** | 53.47 | 92.31 | 1.7 |
| **Heart** | 1.80 | 2.33 | 1.3 |
| **Kidney** | 0.98 | 0.88 | 0.9 |
| **Lung** | 1.54 | 1.62 | 1.0 |
| **Liver** | 4.36 | 1.02 | 0.2 |
| **Spleen** | 2.78 | 2.64 | 1.0 |
| **Small Intestine** | 1.69 | 0.92 | 0.5 |
| **Small Intestinal Wash** | 36.66 | 14.70 | 0.4 |
| **Large Intestine** | 1.08 | 1.01 | 0.9 |
| **Large Intestinal Wash** | 6.46 | 21.18 | 3.3 |
| **Cecum** | 20.20 | 12.72 | 0.6 |
| **Stomach** | 5.34 | 1.83 | 0.3 |
| **Urine** | 1.79 | 3.28 | 1.8 |
| **Faeces** | 4.58 | 0.99 | 0.2 |

### Example 9. In vivo biodistribution of [³H]vinblastine (100 ng/kg) in rats when co-administered with xanthan gum (250 mg/kg)

Male Wistar rats (n=3) (average weight 200-300 g; 11-12 weeks old) were given a single dose of [³H]vinblastine in 3 ml (100 ng/kg, ~1.7 µCi) with xanthan gum (250 mg/kg) by oral gavage. Animals were allowed free access to water throughout, but starved overnight before dosing. Food was made available 2 hours later. The rats were kept in metabolic cages to facilitate collection of urine and faeces for measurement of radioactivity and to prevent coprophagia. Animals were sacrificed at different time points (5 and 30 minutes, 1, 5 and 24 hours) using CO₂ and all systemic organs were removed. The GI tract was also excised and separated into stomach, small intestine, cecum and large intestine. Washings of each section were collected too. Blood was obtained by cardiac puncture. Subsequently, organs were homogenised (using a blade homogeniser) and aliquots were taken, and mixed with scintillation liquid. Radioactivity was assessed using a β-counter. To compensate for quenching, all samples after the initial reading were spiked with a known amount of radioactivity and subsequently re-read. The blood volume of the rat was calculated assuming 7.2ml blood/100g rat.

**Results:-** [³H]Vinblastine biodistribution in the presence of xanthan gum is shown in Table 31 In the presence of xanthan gum there is a [³H]vinblastine increase in AUC of x4 in large intestine, x13.9 in large intestinal wash, x5.4 in urine and x1.7 in faeces. No significant [³H]vinblastine accumulation in any primary organs can be detected when dosed in combination with xanthan gum. AUC values are shown in Table 32.

**Table 31.**

| **[**^{**3**}**H]Vinblastine (100 ng/kg) organ biodistribution (% Dose/organ weight) in the presence of 250 mg/kg Xanthan Gum (n=3 ± SEM)** | | | | | |
|---|---|---|---|---|---|
| | **Time= 5 min** | **Time= 30 min** | **Time= 1 h** | **Time= 5 h** | **Time= 24 h** |
| **Blood** | 5.53 ± 1.09 | 1.45 ± 0.10 | 2.35 ± 0.82 | 2.00 ± 0.25 | 2.66 ± 1.30 |
| **Heart** | 0.14 ± 0.03 | 0.20 ± 0.07 | 0.05 ± 0.01 | 0.14 ± 0.5 | 0.03 ± 0.01 |
| **Kidney** | 0.04 ± 0.00 | 0.10 ± 0.06 | 0.02 ± 0.00 | 0.05 ± 0.01 | 0.02 ± 0.00 |
| **Lung** | 0.09 ± 0.05 | 1.09 ± 1.00 | 0.03 ± 0.01 | 0.08 ± 0.01 | 0.04 ± 0.01 |
| **Liver** | 0.08 ± 0.01 | 0.11 ± 0.05 | 0.03 ± 0.00 | 0.05 ± 0.00 | 0.04 ± 0.00 |
| **Spleen** | 0.09 ± 0.03 | 0.15 ± 0.06 | 0.08 ± 0.03 | 0.15 ± 0.02 | 0.06 ± 0.01 |
| **Small Intestine** | 0.22 ± 0.08 | 0.63 ± 0.23 | 0.44 ± 0.14 | 0.09 ± 0.05 | 0.01 ± 0.00 |
| **Small Intestinal Wash** | 1.92 ± 0.98 | 8.54 ± 3.26 | 9.35 ± 3.28 | 1.27 ± 0.51 | 0.03 ± 0.01 |
| **Large Intestine** | 0.03 ± 0.01 | 0.05 ± 0.00 | 0.06 ± 0.04 | 0.35 ± 0.16 | 0.02 ± 0.00 |
| **Large Intestinal Wash** | 0.04 ± 0.01 | 0.08 ± 0.02 | 0.03 ± 0.01 | 7.52 ± 2.86 | 0.32 ± 0.10 |
| **Cecum** | 0.03 ± 0.01 | 0.02 ± 0.00 | 0.02 ± 0.01 | 3.31 ± 0.94 | 0.10 ± 0.04 |
| **Stomach** | 2.21 ± 0.90 | 2.28 ± 0.60 | 1.77 ± 0.77 | 0.21 ± 0.07 | 0.03 ± 0.01 |
| **Urine** | nc | nc | nc | 0.01 ± 0.00 | 0.99 ± 0.40 |
| **Faeces** | nc | 0.08 ± 0.1 | 0.05 ± 0.00 | 0.05 ± 0.01 | 0.74 ± 0.30 |

**Table 32.**

| **Area Under the Curve (% Dose/organ weight, 5min-24 h)of [**^{**3**}**H]Vinblastine (100 ng/kg) (n=3 ± SEM) in control and in presence of xanthan gum** | | | |
|---|---|---|---|
| **Organ** | **AUC Control** | **AUC (+) 250 mg/kg Xanthan Gum** | **Ratio Xanthan Gum/Control** |
| **Blood** | 53.47 | 55.34 | 1.0 |
| **Heart** | 1.80 | 2.13 | 1.2 |
| **Kidney** | 0.98 | 0.93 | 0.9 |
| **Lung** | 1.54 | 1.91 | 1.2 |
| **Liver** | 4.36 | 1.09 | 0.2 |
| **Spleen** | 2.78 | 2.55 | 0.9 |
| **Small Intestine** | 1.69 | 2.45 | 1.5 |
| **Small Intestinal Wash** | 36.66 | 40.29 | 1.1 |
| **Large Intestine** | 1.08 | 4.37 | 4.0 |
| **Large Intestinal Wash** | 6.46 | 89.58 | 13.9 |
| **Cecum** | 20.20 | 39.12 | 1.9 |
| **Stomach** | 5.34 | 8.14 | 1.5 |
| **Urine** | 1.79 | 9.58 | 5.4 |
| **Faeces** | 4.58 | 7.79 | 1.7 |

### Example 10. In vivo biodistribution of [³H]vinblastine (100 ng/kg) in rats when co-administered with polidocanol (1 g/kg)

Male Wistar rats (n=3) (average weight 200-300 g; 11-12 weeks old) were given a single dose of [³H]vinblastine in 3 ml (100 ng/kg, ~1.7 µCi) polidocanol (1 g/kg) by oral gavage. Animals were allowed free access to water throughout, but starved overnight before dosing. Food was made available 2 hours later. The rats were kept in metabolic cages to facilitate collection of urine and faeces for measurement of radioactivity and to prevent coprophagia. Animals were sacrificed at different time points (5 and 30 minutes, 1, 5 and 24 hours) using CO₂ and all systemic organs were removed. The GI tract was also excised and separated into stomach, small intestine, cecum and large intestine. Washings of each section were collected too. Blood was obtained by cardiac puncture. Subsequently, organs were homogenised (using a blade homogeniser) and aliquots were taken, and mixed with scintillation liquid. Radioactivity was assessed using a β-counter. To compensate for quenching, all samples after the initial reading were spiked with a known amount of radioactivity and subsequently re-read. The blood volume of the rat was calculated assuming 7.2ml blood/100g rat.

**Results:-** [³H]Vinblastine biodistribution in the presence of polidocanol is shown in Table 33 In the presence of polidocanol there is a [³H]Vinblastine increase in AUC of x2.2 in small intestine, x2 in large intestine, x6.7 in large intestinal wash, x7.3 in cecum, x14.0 in urine and x4.4 in faeces. No significant [³H]vinblastine accumulation in any primary organs can be detected when dosed in combination with polidocanol except lung and kidney. The dosage technique employed could account for the increased drug presence in the lung, whereas the increase in kidney can be explained by an increase drug stored in the kidneys awaiting to be excreted. AUC values are shown in Table 34.

**Table 33.**

| **[**^{**3**}**H]Vinblastine (100 ng/kg) organ biodistribution (% Dose/organ weight) in the presence of 1 g/kg Polidocanol (n=3 ± SEM)** | | | | | |
|---|---|---|---|---|---|
| | **Time= 5 min** | **Time= 30 min** | **Time= 1 h** | **Time= 5 h** | **Time= 24 h** |
| **Blood** | 2.93 ± 0.58 | 6.40 ± 2.55 | 2.59 ± 0.90 | 1.81 ± 0.28 | 0.9 ± .11 |
| **Heart** | 0.08 ± 0.01 | 0.41 ± 0.23 | 0.15 ± 0.07 | 0.20 ± 0.09 | 0.04 ± 0.00 |
| **Kidney** | 0.03 ± 0.01 | 0.25 ± 0.02 | 0.15 ± 0.05 | 0.11 ± 0.04 | 0.04 ± 0.00 |
| **Lung** | 0.31 ± 0.15 | 0.64 ± 0.13 | 0.18 ± 0.05 | 0.33 ± 0.24 | 0.05 ± 0.00 |
| **Liver** | 0.08 ± 0.02 | 0.29 ± 0.05 | 0.16 ± 0.06 | .13 ± 0.06 | 0.03 ± 0.0 |
| **Spleen** | 0.09 ± 0.02 | 0.34 ± 0.03 | 0.31 ± 0.14 | 0.23 ± 0.06 | 0.13 ± 0.02 |
| **Small Intestine** | 0.1 ± 0.04 | 0.49 ± 0.06 | 0.10 ± 0.01 | 0.26 ± 0.15 | 0.02 ± 0.00 |
| **Small Intestinal Wash** | 1.70 ± 0.89 | 5.20 ± 1.00 | 1.08 ± 0.26 | 2.17 ± 1.06 | 0.09 ± 0.03 |
| **Large Intestine** | 0.02 ± 0.00 | 0.07 ± 0.00 | 0.05 ± 0.01 | 0.14 ± 0.08 | 0.05 ± 0.02 |
| **Large Intestinal Wash** | 0.06 ± 0.01 | 0.28 ± 0.10 | 0.11 ± 0.07 | 1.70 ± 1.16 | 2.43 ± 1.00 |
| **Cecum** | 0.03 ± 0.00 | 0.08 ± 0.02 | 0.03 ± 0.01 | 0.34 ± 0.16 | 0.24 ± 0.08 |
| **Stomach** | 3.85 ± 2.30 | 9.10 ± 2.07 | 4.41 ± 0.41 | 2.02 ± 1.19 | 0.06 ± 0.03 |
| **Urine** | nc | 0.29 ± 0.15 | 0.16 ± 0.01 | nc | 2.58 ± 0.25 |
| **Faeces** | nc | 0.11 ± 0.02 | nc | 0.16 ± 0.01 | 1.94 ± 0.12 |

**Table 34.**

| **Area Under the Curve (% Dose/organ weight, 5min-24 h)of [**^{**3**}**H]Vinblastine (100 ng/kg) (n=3 ± SEM) in control and in presence of polidocanol** | | | |
|---|---|---|---|
| **Organ** | **AUC Control** | **AUC (+) 1 g/kg Polidocanol** | **Ratio Polidocanol/ Control** |
| **Blood** | 53.47 | 38.77 | 0.7 |
| **Heart** | 1.80 | 3.18 | 1.8 |
| **Kidney** | 0.98 | 2.11 | 2.2 |
| **Lung** | 1.54 | 5.09 | 3.3 |
| **Liver** | 4.36 | 2.20 | 0.5 |
| **Spleen** | 2.78 | 4.73 | 1.7 |
| **Small Intestine** | 1.69 | 3.66 | 2.2 |
| **Small Intestinal Wash** | 36.66 | 31.02 | 0.8 |
| **Large Intestine** | 1.08 | 2.21 | 2.0 |
| **Large Intestinal Wash** | 6.46 | 43.01 | 6.7 |
| **Cecum** | 20.20 | 6.26 | 0.3 |
| **Stomach** | 5.34 | 38.72 | 7.3 |
| **Urine** | 1.79 | 25.05 | 14.0 |
| **Faeces** | 4.58 | 20.37 | 4.4 |

## Claims

1. A polysaccharide, surfactant or dendrimer for use to improve the oral bioavailability of a medicinally active substance.

2. A polysaccharide, surfactant or dendrimer for use to inhibit an efflux pump enzyme of the type causing ejection of medicinally active substances from the cell.

3. A use as claimed in claim 2 wherein the efflux pump enzyme is P-glycoprotein (P-gp).

4. A use as claimed in claim 2, wherein said cell is a gut cell.

5. A polysaccharide, surfactant or dendrimer for use to alleviate the multi drug resistance (MDR) effect.

6. The use of a polysaccharide, surfactant or dendrimer in the manufacture of a medicament to improve the oral bioavailability of a medicinally active substance.

7. The use of a polysaccharide, surfactant or dendrimer in the manufacture of a medicament to inhibit an efflux pump enzyme of the type causing ejection of medicinally active substances from the cell.

8. A use as claimed in claim 7, wherein said cell is gut cell.

9. A use as claimed in claim 7, wherein the efflux pump enzyme is P-glycoprotein (P-gp).

10. The use of a polysaccharide, surfactant or dendrimer in the manufacture of a medicament to prevent or alleviate multi-drug resistance (MDR).

11. A use as claimed in any one of the claims 6-10 wherein the polysaccharide comprises D-mannosyluronic acid, L-gulosyluronic, D-glucose and/or D-glucuronic acid monomers and optionally also D-mamose, D-mannuronic acid and/or D-mannose monomers.

12. A use as claimed in any one of the claims 6-11 wherein the polysaccharide comprises carboxylic acid functional groups or salts thereof.

13. A use as claimed in claim 12 wherein the polysaccharide is in the form of an anionic gum such as xanthan gum or gellan gum.

14. A use as claimed in any one of the claims 6-12 wherein the polysaccharide is a gel.

15. A use as claimed in any one of the claims 6-14 wherein the polysaccharide has a molecular weight of at least 1 x 10⁴ Da, preferably at least 1 x 10⁵ Da, for example from approximately 0.1 x 10⁶ Da to 1 x 10⁷ Da, for example 0.5 x 10⁶ Da to 2 x 10⁶ Da.

16. A use as claimed in any one of the claims 6-15 wherein the polysaccharide is used in an amount of from 5 mg dose/kg body weight/day to about 5 g dose/kg body weight/day.

17. A use as claimed in any one of claims 6-10 wherein the surfactant is a polyoxyethylene alkyl ether such as an ether of the formula I:
Alk ― (OCH₂CH₂)ₙ-OH (I)
wherein n is an integer from 5 to 16 and Alk is a C₅₋₁₈ alkyl group.

18. A use as claimed in claim 17 wherein the surfactant is a compound of formula I wherein n is 9 and Alk is a lauryl (C₁₂ alkyl) group.

19. A use as claimed in any one of claims 6-10 wherein the dendrimer is described by of the fomula II: 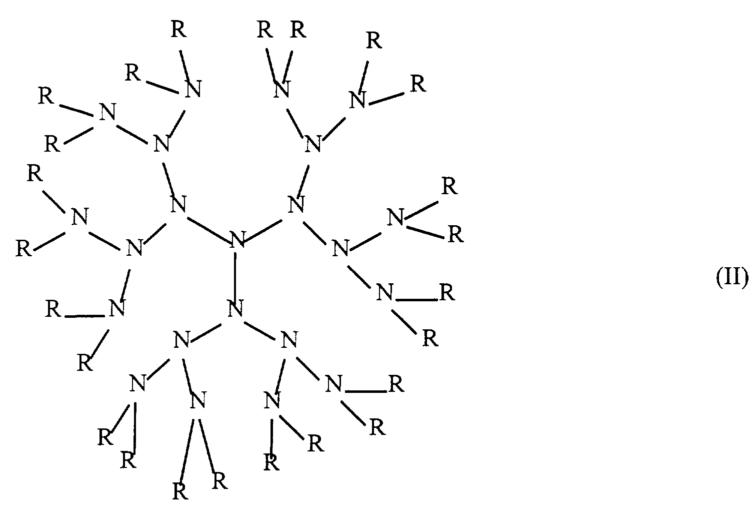 (or a pharmaceutically acceptable salt thereof) wherein each R moiety is independently hydrogen, an NH ₂ group or a -COOH group.

20. A use as claimed in any one of claims 6-10 wherein the dendrimer is described by of the fomula III: 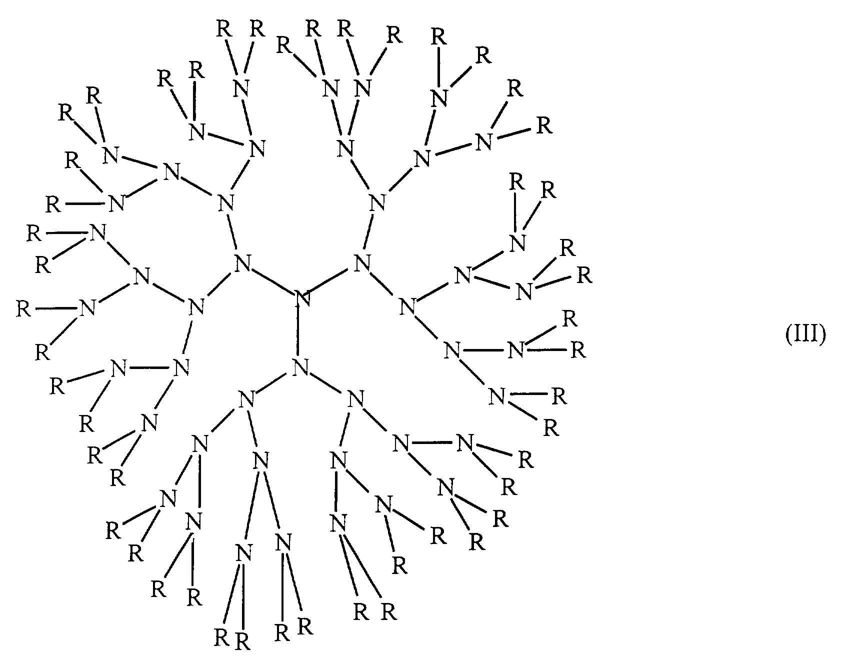 (or a pharmaceutically acceptable salt thereof) wherein each R moiety is independently hydrogen an -NH₂ group or a or a -COOH group.

21. A use as claimed in any one of claims 6-10 wherein the dendrimer is a generation 3 cationic dendrimer of the formula IV: 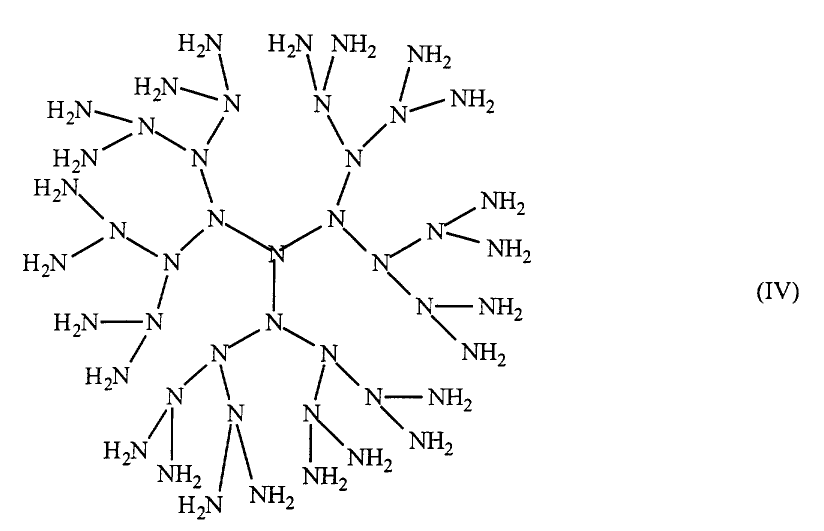 or a pharmaceutically acceptable salt thereof.

22. A use as claimed in any one of the claims 6-21wherein the medicinally active substance is a compound which is a substrate for an efflux pump system of the type causing ejection of medicinally active substances from the cell.

23. A use as claimed in any one of the claims 6-22 wherein the medicinally active substance is a substrate for P-glycoprotein (P-gp), or MRP.

24. A use as claimed in any one of the claims 6-23wherein the medicinally active substance is an anti-tumour or anti-neoplastic agent.

25. A use as claimed in any one of the claims 6-23 wherein the medicinally active substance is an antibiotic/antibacterial agent.

26. A use as claimed in any one of the claims 6-23 wherein the medicinally active substance is an antiviral agent.

27. A use as claimed in any one of the claims 6-23 wherein the medicinally active substance is an antifungal agent.

28. A use as claimed in any one of the claims 6-23 wherein the medicinally active substance is an antidepressant agent.

29. A pharmaceutical composition for oral administration comprising a medicinally active substance and an amount of a polysaccharide effective to increase the oral bioavailability of the active substance.

30. A pharmaceutical composition for oral administration comprising a medicinally active substance and an amount of a polysaccharide effective to inhibit CYP3A, P-glycoprotein (P-gp), or MRP.

31. A pharmaceutical composition as claimed in claim 29 or claim 30 wherein the polysaccharide comprises D-mannosyluronic acid, L-gulosyluronic, D-glucose and/or D-glucuronic acid monomers and optionally also D-mamose, D-mannuronic acid and/or D-mannose monomers.

32. A pharmaceutical composition as claimed in any one of claims 29 to 31 wherein the polysaccharide comprises carboxylic acid functional groups or salts thereof.

33. A pharmaceutical composition as claimed in any one of claims 29 to 32 wherein the polysaccharide is an anionic gum.

34. A pharmaceutical composition as claimed in claim 33 wherein the polysaccharide is xanthan gum or gellan gum.

35. A pharmaceutical composition as claimed in any one of claims 29 to 32 wherein the polysaccharide is a gel.

36. A pharmaceutical composition as claimed in any one of claims 29 to 35 wherein the polysaccharide has a molecular weight of from approximately 0.1 x 10⁶ Da to 1 x 10⁷ Da.

37. A pharmaceutical composition for oral administration comprising a medicinally active substance and an amount of a surfactant in the form of a polyoxyethylene alkyl ether such as an ether of the formula I:
Alk―(OCH₂CH₂)ₙ-OH (I)
wherein n is an integer from 5 to 16 and Alk is a C₅₋₁₈ alkyl group, effective to increase the bioavailability of the active substance.

38. A pharmaceutical composition for oral administration comprising a medicinally active substance and an amount of a surfactant in the form of a polyoxyethylene alkyl ether of the formula I:
Alk―(OCH₂CH₂)ₙ-OH (I)
wherein n is an integer from 5 to 16 and Alk is a C₅₋₁₈ alkyl group, effective to inhibit CYP3A, P-glycoprotein (P-gp) or MRP.

39. A pharmaceutical composition as claimed in claim 37 or claim 38 wherein the surfactant is a compound of formula I wherein n is 9 and Alk is a lauryl (C12 alkyl) group.

40. A pharmaceutical composition for oral administration comprising a medicinally active substance and an amount of a dendrimer of the fomula II: 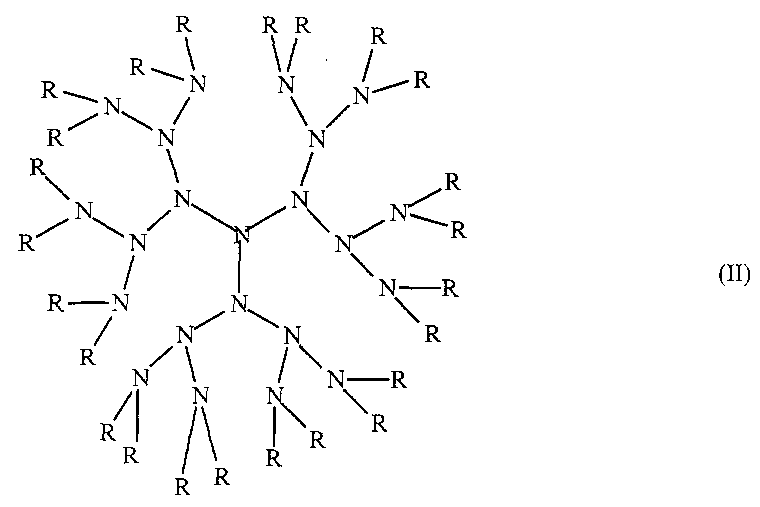 (or a pharmaceutically acceptable salt thereof) or a compound of the formula III: 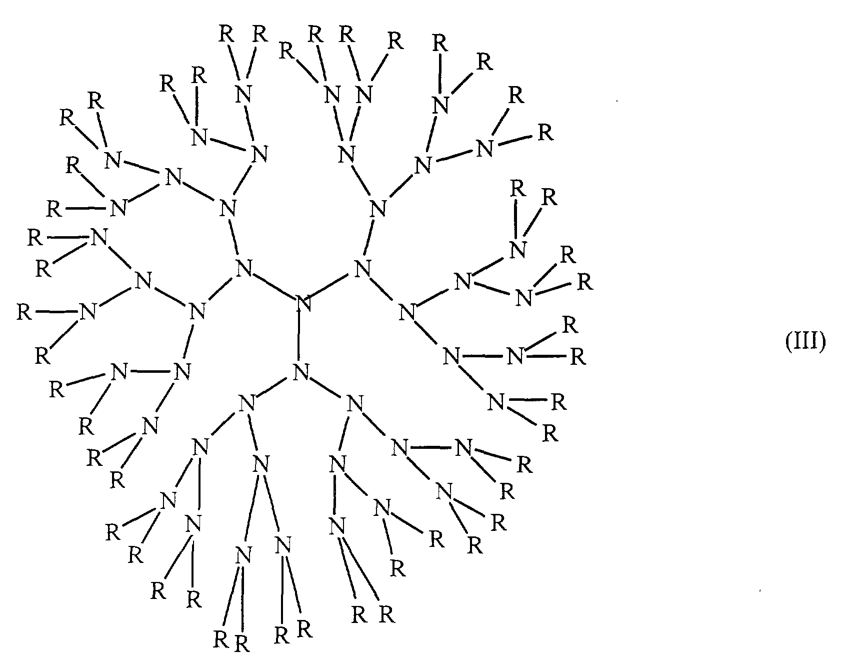 (or a pharmaceutically acceptable salt thereof) wherein each R moiety is independently hydrogen, an - NH₂ group or a -COOH group, effective to increase the bioavailability of the active substance.

41. A pharmaceutical composition for oral administration comprising a medicinally active substance and an amount of a dendrimer of the fomula II: 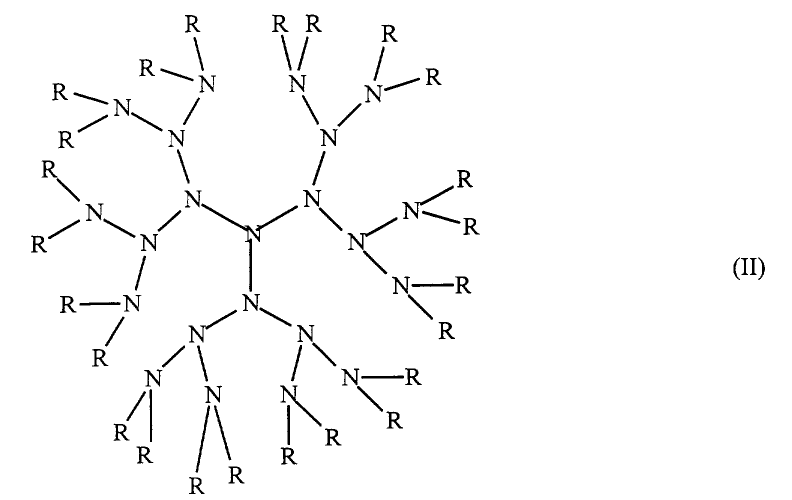 or a compound of the formula III: 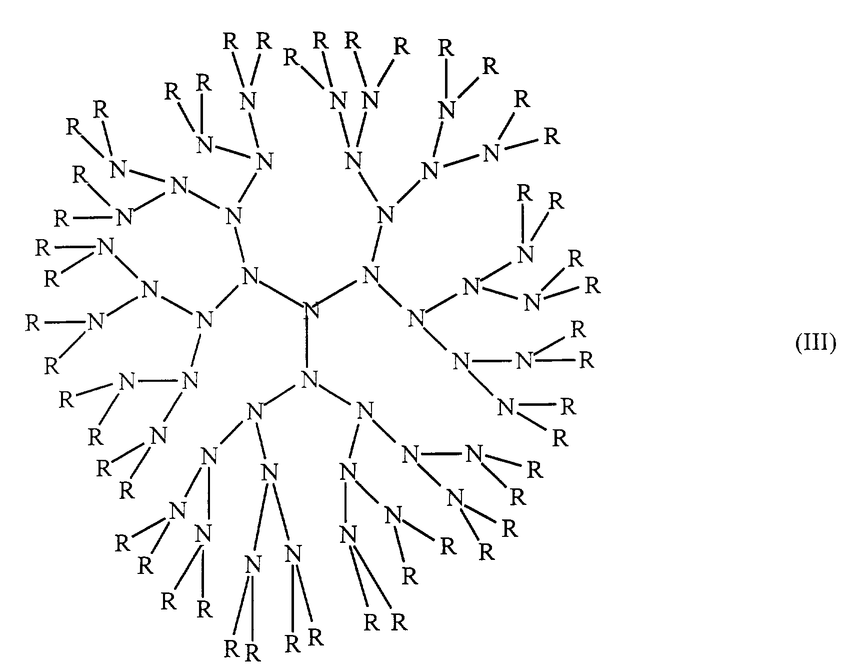 wherein each R moiety is independently hydrogen an -NH₂ group or a -COOH group, effective to inhibit CYP3A, P-glycoprotein (P-gp) or MRP.

42. A pharmaceutical composition as claimed in claim 40 or claim 41 wherein the bioavailability enhancer is a generation 3 cationic dendrimer of the formula IV: 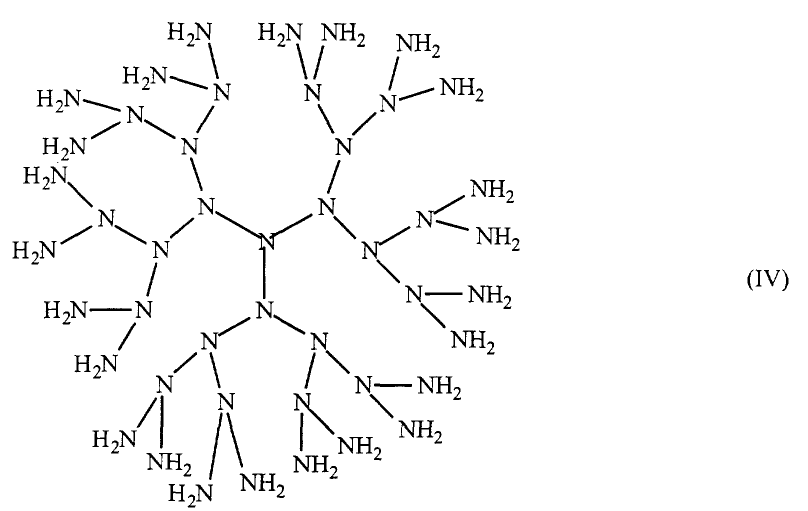 (or a pharmaceutically acceptable salt thereof).

43. A pharmaceutical composition as claimed in any one of claims 22 to 42 wherein the medicinally active substance is a compound which is a substrate for an efflux pump system of the type causing ejection of medicinally active substances from the cell.

44. A pharmaceutical composition as claimed in any one of claims 29 to 42 wherein the medicinally active substance is a substrate for P-glycoprotein (P-gp) or MRP.

45. A pharmaceutical composition as claimed in any one of claims 29 to 42 wherein the medicinally active substance is an anti-tumour or anti-neoplastic agent.

46. A pharmaceutical composition as claimed in any one of claims 29 to 42 wherein the medicinally active substance is an antibiotic/antibacterial agent.

47. A pharmaceutical composition as claimed in any one of claims 29 to 42 wherein the medicinally active substance is an antiviral agent.

48. A pharmaceutical composition as claimed in any one of claims 29 to 42 wherein the medicinally active substance is an antifungal agent.

49. A pharmaceutical composition as claimed in any one of claims 29 to 42 wherein the medicinally active substance is an antidepressant agent.
